# EUROPEAN PATENT SPECIFICATION

(11) **EP 3 993 736 B1**
(45) Date of publication and mention of the grant of the patent: **15.11.2023**
(21) Application number: 20745377.0
(22) Date of filing: 30.06.2020
(51) Int. Cl.: A61F 2/24

(54) **PROSTHETIC HEART VALVE AND DELIVERY APPARATUS THEREFOR**
HERZKLAPPENPROTHESE UND FREISETZUNGSVORRICHTUNG DAFÜR
VALVULE CARDIAQUE PROTHÉTIQUE ET APPAREIL DE POSE ASSOCIÉ

(30) Priority: 02.07.2019 US 201962869948 P
(43) Date of publication of application: 11.05.2022
(73) Proprietor: Edwards Lifesciences Corporation, Irvine, CA 92614-5688 (US)
(72) Inventor: LEVI, Tamir S., 30889 Caesarea (IL); BUKIN, Michael, 30889 Caesarea (IL); NIR, Noam, 30889 Caesarea (IL); YOHANAN, Ziv, 30889 Caesarea (IL); SCHWARCZ, Elazar Levi, 30889 Caesarea (IL); COHEN, Oren, 30889 Caesarea (IL); WITZMAN, Ofir, 30889 Caesarea (IL); ATIAS, Eitan, 30889 Caesarea (IL); MILLER, Noam, 30889 Caesarea (IL); PERLMUTTER, Khen, 3055704 Binyamina (IL); MANASH, Boaz, 3052602 Givat Ada (IL); LEIBA, Eyal, 32022 N. Misgav (IL); GOLDBERG, Eran, 30889 Caesarea (IL)
(74) Representative: Eisenführ Speiser
(86) International application number: PCT/US2020/040318
(87) International publication number: WO 2021/003167

(56) References cited:
- US-A1- 2014 296 962

## Description

### CROSS REFERENCE TO RELATED APPLICATION

This application claims the benefit of U.S. Application No. 62/869,948, filed July 2, 2019.

### FIELD

The present disclosure relates to implantable, mechanically expandable prosthetic devices, such as prosthetic heart valves, and to methods and delivery apparatus for implanting such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

Prosthetic heart valves that rely on a mechanical actuator for expansion can be referred to as "mechanically expandable" prosthetic heart valves. Mechanically expandable prosthetic heart valves can provide one or more advantages over self-expandable and balloon-expandable prosthetic heart valves. For example, mechanically expandable prosthetic heart valves can be expanded to various diameters. Mechanically expandable prosthetic heart valves can also be compressed after an initial expansion (e.g., for repositioning and/or retrieval).

Despite these advantages, mechanically expandable prosthetic heart valves can present several challenges. For example, it can be difficult to control expansion and contraction of a mechanically expandable prosthetic heart valve and/or to lock the prosthetic heart valve in the desired configuration. It can also be difficult to attach a valve structure to a frame of a mechanically expandable prosthetic heart valve. Accordingly, there is a need for improved mechanically expandable prosthetic heart valves, as well as delivery apparatus and methods.

### SUMMARY

Described herein are prosthetic heart valves, delivery apparatus, and methods for implanting prosthetic heart valves. The disclosed devices and methods can, for example, provided controlled and predictable expansion and contraction of a mechanically expandable prosthetic heart valve and the ability to lock the prosthetic heart valve in the desired configuration. The disclosed devices and methods can also facilitate mounting a valve structure to a frame of a mechanically expandable prosthetic heart valve in a simple and reliable way.

The present invention relates to a prosthetic heart valve as defined in independent claim 1. The prosthetic heart valve comprises a frame, a valve structure, and an actuator. The frame comprises a plurality struts and having an inflow end, an outflow end, and a longitudinal axis extending from the inflow end to the outflow end. The struts are pivotably coupled together such that the frame can pivot between one or more radially compressed states and one or more radially expanded states. The valve structure is disposed within the frame and comprises a plurality of leaflets configured to allow blood flow through the valve structure from the inflow end of the frame to the outflow end of the frame and to restrict blood flow through the valve structure from the outflow end of the frame to the inflow end of the frame. The actuator coupled to the frame and configured to move the frame between the one or more radially compressed states and the one or more radially expanded states. The actuator comprises a first portion, a second portion, a first lumen, a second lumen, a locking member, and a locking element. The first lumen and the second lumen extend axially through the first portion and the second portion in a direction parallel to the longitudinal axis of the frame. The first lumen and the second lumen are spaced apart from each other and are parallel to each other. The locking member is disposed in the first lumen, is fixedly coupled to the first portion, and is axially movable relative to a second portion. The second lumen is configured to receive an actuation shaft of a delivery apparatus. The actuation shaft can be releasably coupled to the first portion and can move axially relative to the second portion, and thereby can move the frame between the one or more radially compressed states and the one or more radially expanded states. The locking element is adjustably coupled to the locking member and configured to restrict relative axial movement between the locking member and the second portion, and thereby lock the frame in the one or more radially compressed states or the one or more radially expanded states.

In some embodiments, the first portion of the actuator is a distal support member, and the second portion of the actuator is an intermediate support member.

In some embodiments, the locking element is a locking nut, and the locking nut is adjustably coupled to the locking member by a threaded connection.

In some embodiments, the actuator further comprises a third portion, and the third portion is a proximal support member.

In some embodiments, the prosthetic heart valve comprises exactly one actuator.

In some embodiments, the actuator is one of a plurality of actuators, and the actuators are spaced apart circumferentially relative to each other.

In some embodiments, the prosthetic heart valve comprises exactly three actuators.

In some embodiments, the actuator comprises a window disposed between the first lumen and the second lumen, and the window is configured to receive a commissure of the leaflets of the valve structure.

In some embodiments, the actuator comprises an open slot disposed between the first lumen and the second lumen, and the open slot is configured to receive a preassembled commissure of the leaflets.

In some embodiments, the frame comprises an apex formed by a pair of struts that is disposed adjacent to the actuator, and the pair of struts comprises a first segment that extends radially outwardly relative to a main body of the frame and relative to the actuator such that there is a radial gap between the first segment and the actuator.

In some embodiments, the pair of struts comprises a second segment extending axially from the first segment toward the outflow end of the frame and extending radially inwardly from the first segment so as to radially overlap with an outflow end of the actuator.

Also disclosed herein is a delivery assembly which comprises a prosthetic heart valve and a delivery apparatus. The delivery apparatus comprises an actuation shaft and a locking shaft. The actuation shaft is configured to be releasably coupled to the first portion of the actuator and to move axially relative to the second portion of the actuator, and thereby move the frame between the one or more radially compressed states and the one or more radially expanded states. The locking shaft is adjustably coupled to the locking member and configured to move the locking element relative to the locking member.

Also disclosed herein is a prosthetic heart valve which comprises a mechanically expandable frame, a valve structure, and an actuator. The frame comprises a plurality of interconnected struts. The frame is movable from a radially compressed state and a radially expanded state and movable from the radially expanded state to the radially compressed state. The valve structure is disposed radially within the frame and comprising a plurality of leaflets. The actuator is coupled to the frame and comprises an actuation lumen, a locking member, and a locking element. The actuation lumen is circumferentially spaced and axially parallel to the locking member. The actuation lumen is configured for receiving an axially movable actuation shaft that can move the frame between the radially compressed state and the radially expanded state. The locking element is rotatably coupled to the locking member and is configured to lock the frame at a desired radially compressed state or a desired radially expanded state.

In some implementations, the actuator comprises a first support member, a second support member, and a third support member. The actuation lumen extends from the first support member, through the second support member, and through the third support member.

In some implementations, the locking member extends from the first support member, through second support member, and into the third support member.

In some implementations, the locking member comprises external threads, and the locking element comprises internal threads configured to threadably mate with the external threads of the locking member.

Also disclosed herein is a method of implanting a prosthetic heart valve. The method comprises inserting a prosthetic heart valve into a patient's vasculature, and the prosthetic heart valve is in a radially compressed delivery configuration and coupled to a delivery apparatus. The method further comprises positioning the prosthetic heart valve at or near an implantation location by manipulating the delivery apparatus, expanding the prosthetic heart valve from the radially compressed delivery configuration to a radially expanded functional configuration by moving a first shaft of the delivery apparatus in a first axial direction relative to an actuator of the prosthetic heart valve, and locking the prosthetic heart valve at the radially expanded functional configuration by rotating a second shaft of the delivery apparatus in a first rotational direction relative to the actuator. The second shaft is spaced circumferentially and parallel relative to the first shaft.

In some implementations, prior to the act of locking the prosthetic heart valve, the method further comprises compressing the prosthetic heart valve from the radially expanded functional configuration to an intermediate configuration by moving the first shaft of the delivery apparatus in a second axial direction relative to the actuator.

Also disclosed herein is a prosthetic heart valve which comprises a frame, a valve structure, and an actuator. The frame comprises a plurality of interconnected struts, and the frame is radially expandable and radially compressible and has a first end portion and a second end portion. The valve structure disposed radially within the frame and comprising a plurality of leaflets. The actuator coupled to the frame and configured to control radial expansion and radial compression of the frame and to selectively secure the frame at a desired radial configuration. The actuator comprises an actuation plate and a locker housing, and the actuation plate is coupled to the first end portion of the frame. The locker housing is coupled the second end portion of the frame. The actuation plate extends from the first end portion of the frame and into the locker housing. The actuation plate is configured to selectively engage the locker housing. When the actuation plate is engaged with the locker housing, relative movement between the actuation plate and the locker housing is restricted, thereby securing the frame a desired radial configuration, and when the actuation plate is disengaged from the locker housing, the actuation plate can move axially relative to the locker housing, thereby allowing radial expansion and radial compression of the frame.

In some implementations, the actuation plate comprises jaws that are movable between an open state and a closed state. In the open state, the jaws are configured to engage the locker housing, and in the closed state, the jaws are configured to disengage the locker housing.

In some implementations, the jaws of the actuation plate are biased to the open state.

In some implementations, the locker housing has a circular cross-sectional profile taken in a plane perpendicular to a longitudinal axis of the locker housing.

In some implementations, the locker housing has a U-shaped cross-sectional profile taken in a plane perpendicular to a longitudinal axis of the locker housing.

In some implementations, the locker housing has a rectangular cross-sectional profile taken in a plane perpendicular to a longitudinal axis of the locker housing.

In some implementations, the locker housing comprises a plurality of slots, and the slots are axially spaced relative to each other and are configured to receive the actuation plate.

In some implementations, the actuation plate comprises tabs configured to extend into the slots of the locker housing when the actuation plate is engaged with the locker housing.

In some implementations, the slots of the locker housing comprises a first column of slots and a second column of slots, each column of slots extends axially, and the first column of slots is axially offset relative to the second column of slots.

In some implementations, the slots of the locker housing extend at an angle less than 90 degrees relative to a longitudinal axis of the locker housing.

Also disclosed herein is a delivery assembly which comprises a prosthetic heart valve and a delivery apparatus. The delivery apparatus comprises an actuation shaft and a locking shaft. The actuation shaft is configured to be releasably coupled to the first portion of the actuator and to move axially relative to the second portion of the actuator, and thereby move the frame between the one or more radially compressed states and the one or more radially expanded states. The locking shaft is adjustably coupled to the locking member and configured to move the locking element relative to the locking member.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 depicts a perspective view of a mechanically expandable prosthetic heart valve, according to one embodiment.
FIG. 2 depicts a side view of the frame of the mechanically expandable prosthetic heart valve of FIG. 1, with the frame shown in a compressed or delivery configuration.
FIG. 3 depicts a perspective view of another exemplary mechanically expandable prosthetic heart valve, without the valve structure.
FIG. 4 depicts a perspective view of the mechanically expandable prosthetic heart valve of FIG. 3, with the frame shown in a compressed configuration.
FIG. 5 depicts a detail view of an actuator and an interior side of the frame of the prosthetic heart valve of FIG. 3.
FIG. 6 depicts a detail view of the actuator and an exterior side of the frame of the prosthetic heart valve of FIG. 3.
FIG. 7 depicts the prosthetic heart valve as shown in FIG. 5, with the actuator shown in partial cross section.
FIG. 8 depicts a schematic representation of a delivery apparatus.
FIG. 9 depicts a detail view of distal end portions of shafts of the delivery apparatus of FIG. 8.
FIG. 10 depicts a detail view of the delivery apparatus of FIG. 8 coupled to the prosthetic heart valve of FIG. 3.
FIG. 11 depicts a perspective view of the delivery apparatus of FIG. 8 coupled to the prosthetic heart valve of FIG. 3, with prosthetic heart valve shown in a radially expanded configuration.
FIG. 12 depicts a perspective view of the delivery apparatus of FIG. 8 coupled to the prosthetic heart valve of FIG. 3, with prosthetic heart valve shown in a radially compressed configuration.
FIGS. 13-16 depict the prosthetic heart valve of FIG. 3 being implanted within a native aortic valve of a heart with the delivery apparatus of FIG. 8, the heart shown in partial cross section.
FIG. 17 depicts a perspective view of another exemplary mechanically expandable prosthetic heart valve.
FIG. 18 depicts a detail view of an actuator and an interior side of the frame of the prosthetic heart valve of FIG. 17.
FIG. 19 depicts a detail view of a valve structure attached to the actuator of the prosthetic heart valve of FIG. 17.
FIG. 20 depicts a cross-sectional view of the actuator of the prosthetic heart valve of FIG. 17, with the valve structure attached thereto.
FIG. 21 depicts a partial plan view of the outflow end portion of the prosthetic heart valve of FIG. 17.
FIG. 22 depicts a perspective view a proximal support member of a prosthetic heart valve.
FIG. 23 depicts a partial perspective view of a valve structure being coupled to the proximal support member of FIG. 22.
FIG. 24 depicts a perspective view of another exemplary mechanically expandable prosthetic heart valve.
FIG. 25 depicts a partial side view of the prosthetic heart valve of FIG. 24.
FIG. 26 depicts a perspective view of another exemplary mechanically expandable prosthetic heart valve.
FIG. 27 depicts a side view of an actuation plate of the prosthetic heart valve of FIG. 26, showing jaws of the actuation plate in an open configuration.
FIG. 28 depicts a detail of the actuation plate of the prosthetic heart valve of FIG. 26, showing the jaws of the actuation plate in a closed configuration.
FIG. 29 depicts a side view of a locker housing of the prosthetic heart valve of FIG. 26.
FIG. 30 depicts a perspective view of another exemplary delivery apparatus.
FIG. 31 depicts a partial perspective view of a distal end portion of the delivery apparatus of FIG. 30.
FIG. 32 depicts a partial side view of an actuation shaft of the delivery apparatus of FIG. 30 and the actuation plate of the prosthetic heart valve of FIG. 26, showing the jaws of the actuation plate in the open configuration.
FIG. 33 depicts a partial side view of the actuation shaft of the delivery apparatus of FIG. 30 and the actuation plate of the prosthetic heart valve of FIG. 26, showing the jaws of the actuation plate in the closed configuration.
FIG. 34 depicts a partial side view of a locking shaft of the delivery apparatus of FIG. 30 and the actuation plate of the prosthetic heart valve of FIG. 26, showing the jaws of the actuation plate disposed within the locking shaft and being retained in the closed configuration by the locking shaft.
FIG. 35 depicts a detail view of the prosthetic heart valve of FIG. 26 and the delivery apparatus of FIG. 30, with an actuator of the prosthetic heart valve in a locked state and released from the delivery apparatus, and with the locker housing of the prosthetic heart valve and the locking shaft of the delivery apparatus shown in cross section.
FIG. 36 depicts a detail view of the prosthetic heart valve of FIG. 26 and the delivery apparatus of FIG. 30, with the actuator of the prosthetic heart valve in an unlocked state and coupled to the delivery apparatus, and with the locker housing of the prosthetic heart valve and the locking shaft of the delivery apparatus shown in cross section.
FIG. 37 depicts an end view of the outflow end portion of the prosthetic heart valve of FIG. 26.
FIG. 38 depicts a partial perspective view of a handle of the delivery apparatus of FIG. 30.
FIG. 39 depicts an exploded view of a portion of the handle of the delivery apparatus of FIG. 30.
FIG. 40 depicts a side view of a first side of a locker housing for a prosthetic heart valve.
FIG. 41 depicts a side view of a second side of a locker housing for a prosthetic heart valve.
FIG. 42 depicts an end view of the outflow end portion of the prosthetic heart valve of FIG. 26 with the locker housing of FIG. 40.
FIG. 43 depicts a cross-sectional view of a locking shaft of a delivery apparatus together with the locker housing of FIG. 40, the actuation plate of the prosthetic heart valve of FIG. 26, and the actuation shaft of the delivery apparatus of FIG. 30, showing the jaws of the actuation plate in the unlocked configuration and secured to the actuation shaft.
FIG. 44 depicts a cross-sectional view of the locking shaft of FIG. 43 together with the locker housing of FIG. 40, the actuation plate of the prosthetic heart valve of FIG. 26, and the actuation shaft of the delivery apparatus of FIG. 30, showing the jaws of the actuation plate in the locked configuration and released from the actuation shaft.
FIG. 45 depicts a side view of the locker housing of FIG. 40 and the actuation plate of the prosthetic heart valve of FIG. 26, showing the jaws of the actuation plate in the locked configuration.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Exemplary Embodiments

Described herein are exemplary mechanically expandable prosthetic heart valves, delivery apparatus, and methods of implantation. The disclosed mechanically expandable prosthetic heart valves and methods can, for example, provide controlled and predictable expansion and contraction of the mechanically expandable prosthetic heart valve and provide the ability to lock the prosthetic heart valve in the desired configuration. The disclosed devices and methods can also facilitate mounting a valve structure to a frame of a mechanically expandable prosthetic heart valve in a simple and reliable manner.

As mentioned above, the present invention is defined by independent claim 1. Preferred configurations of the claimed invention are defined in dependent claims 2 to 12. In so far as any of the embodiments or examples described hereafter are not encompassed by the scope of the claims, they are considered to be as supplementary background information and do not constitute a definition of the claimed invention per se.

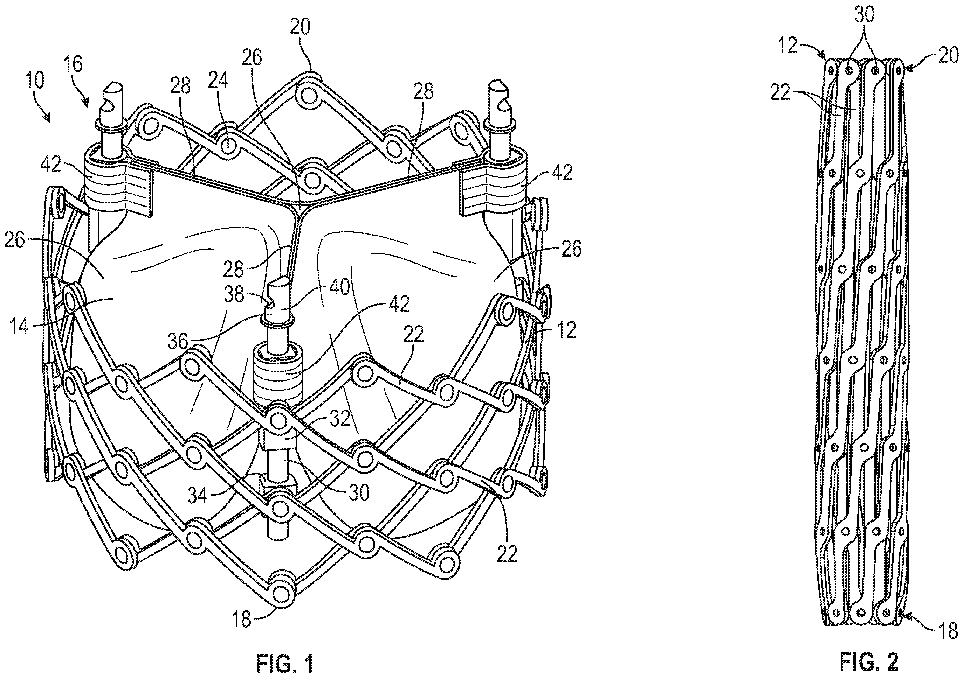

FIG. 1 shows a prosthetic heart valve 10, according to one embodiment. In the illustrated embodiment, the prosthetic valve 10 is configured to be implanted within a native aortic valve. In other embodiments, the prosthetic valve 10 can be configured to be implanted at other locations in the heart, including within a native mitral valve, a native pulmonary valve, and/or a native tricuspid valve.

The prosthetic valve 10 comprises three main components: an annular stent or frame 12, a valve structure 14, and one or more actuators (e.g., three actuators 16 in the illustrated embodiment). The frame 12 is configured for supporting the valve structure 14 and for securing the prosthetic valve 10 within a native heart valve. The valve structure 14 is coupled to the frame 12 and/or to the actuators 16 and is configured to allow blood to flow through the prosthetic valve 10 in one direction. The actuators 16 are coupled to the frame 12 and configured to adjust expansion of the frame 12 to a plurality of configurations including one or more functional or expanded configurations (e.g., FIG. 1), one or more delivery or compressed configurations (e.g., FIG. 2), and/or one or more intermediate configurations. It should be noted that only the frame 12 of the prosthetic valve 10 is shown in FIG. 2.

Referring to FIG. 1, the frame 12 of the prosthetic valve 10 has a first end 18 and a second end 20. In the illustrated embodiment, the first end 18 of the frame 12 is an inflow end and the second end 20 of the frame 12 is an outflow end. In other embodiments, the first end 18 of the frame 12 can be the outflow end and the second end 20 of the frame 12 can be the inflow end.

The frame 12 of the prosthetic valve 10 can be made of any of various suitable materials, including biocompatible metals and/or biocompatible polymers. Exemplary biocompatible metals from which the frame can be formed include stainless steel, cobalt chromium alloy, and/or nickel titanium alloy (which can also be referred to as "NiTi" or "nitinol"). Referring still to FIG. 1, the frame 12 includes a plurality of interconnected struts 22 arranged in a lattice-type pattern. The struts 22 are shown in FIG. 1 as positioned diagonally (or offset at an angle relative to and radially offset from) a longitudinal axis of the prosthetic valve 10 when the prosthetic valve 10 is in the expanded configuration. In other configurations, one or more of the struts 22 can be offset by a different amount than the amount depicted in FIG. 1. Also, as shown in FIG. 2, one or more of the struts 22 can be positioned parallel (or at least substantially parallel) to the longitudinal axis of the prosthetic valve 10.

To facilitate movement between the expanded and compressed configurations, the struts 22 are pivotably coupled to one another at one or more pivot joints along the length of each strut. For example, each of the struts 22 can be formed with apertures at opposing ends and along the length of the strut. The frame 12 comprises hinges at the locations where struts 22 overlap and are pivotably coupled together via fasteners such as rivets or pins 24 that extend through the apertures of the struts. The hinges allow the struts 22 to pivot relative to one another as the frame 12 moves between the radially expanded and compressed configurations, such as during assembly, preparation, and/or implantation of the prosthetic valve 10.

In some embodiments, the frame 12 can be constructed by forming individual components (e.g., the struts 22 and pins 24 of the frame 12) and then mechanically assembling and coupling the individual components together. In other embodiments, the struts are not coupled to each other with respective hinges but are otherwise pivotable or bendable relative to each other to permit radial expansion and contraction of the frame. For example, a frame can be formed (e.g., via laser cutting, electroforming or physical vapor deposition) from a single piece of material (e.g., a metal tube). Further details regarding the construction of frames and prosthetic valves are described in U.S. Patent No. 10,603,165, U.S. Publication Nos. 2018/0344456, and 2019/0060057, and International Publication No. WO 2020/081893. Additional examples of expandable prosthetic valves that can be used with the delivery apparatuses disclosed herein are described in U.S. Patent Nos. 9,700,442 and 9,827,093.

The valve structure 14 of the prosthetic valve 10 is coupled to the frame 12 and configured to regulate the flow of blood through the prosthetic valve 10 from the inflow end 18 to the outflow end 20. The valve structure 14 can include, for example, a leaflet assembly comprising one or more (e.g., three) leaflets 26 made of a flexible material. The leaflets 26 of the leaflet assembly can be made from in whole or part, biological material, biocompatible synthetic materials, or other such materials. Suitable biological material can include, for example, bovine pericardium (or pericardium from other sources). The leaflets 26 can be arranged to form commissures 28 (e.g., pairs of adjacent leaflets), which can, for example, be mounted to respective actuators 16. Further details regarding transcatheter prosthetic heart valves, including the manner in which the valve structure 14 can be coupled to the frame 12 of the prosthetic valve 10, can be found below with reference to other embodiment and/or in U.S. Patent Nos. 6,730,118, 7,393,360, 7,510,575, 7,993,394, and 8,652,202, and U.S. Publication No. 2018/0325665.

The actuators 16 of the prosthetic valve 10 are configured to radially expand and compress the frame 12. For this reason, the actuators 16 can also be referred to as "expansion mechanisms." The actuators 16 are mounted to and spaced around the inner surface of the frame 12. Each of the actuators 16 can be configured to form a releasable connection with one or more respective actuators of a delivery apparatus, as further described below.

Each of the actuators 16 comprises a screw or threaded rod 30, a first anchor 32 (e.g., a cylinder or sleeve), and a second anchor 34 (e.g., a threaded nut). The rod 30 extends through the first and second anchors 32, 34. The first and second anchors 32, 34 can be secured to the frame 12, such as with a respective pin 24 that form hinges at junctions between two struts 22. Each actuator 16 is configured to adjust the distance between the attachment locations of a respective first anchor 32 and second anchor 34. Rotating an actuator in a first direction relative to a respective first and second anchor increases the distance between the attachment locations of the respective first and second anchor and causes the frame to elongate axially and compress radially (e.g., FIG. 2). Rotating the actuator in a second direction relative to the respective first and second anchor decreases the distance between the attachment locations of the respective first and second anchor and causes the frame to foreshorten axially and expand radially (e.g., FIG. 1).

For example, each rod 30 can have external threads that engage internal threads of the second anchor 34 such that rotation of the rod 30 causes corresponding axial movement of the first anchor 32 toward or away from the second anchor 34 (depending on the direction of rotation of the rod 30). This causes the hinges supporting the first and second anchors 32, 34 to move closer towards each other to radially expand the frame 12 or to move farther away from each other to radially compress the frame 12 (depending on the direction of rotation of the rod 30).

In other embodiments, the actuators can be linear type actuators configured to apply axial directed forces to the frame to produce radial expansion and compression of the frame. For example, the rod of each actuator can be fixed relative one of the anchors and slidable the other anchor. For example, the rod can be fixed relative to the second anchor and slidable relative to the first anchor. Thus, in this manner, moving the rod distally relative to the first anchor and/or moving the first anchor proximally relative to the rod radially compresses the frame. Conversely, moving the rod proximally relative to the first anchor and/or moving the first anchor distally relative to the rod radially expands the frame.

When reciprocating type actuators are used, the prosthetic valve can also include one or more locking mechanisms that retain the frame in the expanded state. The locking mechanisms can be separate components that are mounted on the frame apart from the actuators, or they can be a sub-component of the actuators themselves. In particular embodiments, the actuators can comprise combination expansion and locking mechanism as further described below and/or in U.S. Publication No. 2018/0153689.

Each rod 30 can include an attachment member 36 at a proximal end portion of the rod 30. The attachment member can be configured to form a releasable connection with a corresponding actuator of a delivery apparatus. The actuator(s) of the delivery apparatus can apply forces to the rods 30 of the prosthetic valve 10 to radially compress or expand the prosthetic valve 10. The attachment member 36 in the illustrated embodiment comprises a notch 38 and a projection 40 that can engage with corresponding structures of an actuator of the delivery apparatus.

In the illustrated embodiment, the prosthetic valve 10 includes three actuators 16, although a greater or fewer number of actuators could be used in other embodiments. For example, in one embodiment, the prosthetic valve can have one actuator.

The leaflets 26 of the valve structure 14 can have commissure attachments members 42 that wrap around the first anchors 32 of the actuators 16. Further details of the actuators, locking mechanisms and delivery apparatuses for actuating the actuators can be found in U.S. Publication Nos. 2018/0153689, 2018/0325665, and 2019/0060057. Any of the actuators and locking mechanisms disclosed in the previously filed applications can be incorporated in any of the prosthetic valves disclosed herein. Also, any of the locking mechanisms disclosed herein can be incorporated in any of the prosthetic valves disclosed in the previously filed applications. Further, any of the delivery apparatuses disclosed in the previously filed applications can be used to deliver and implant any of the prosthetic valves discloses herein, or vice versa.

Although not shown in FIG. 1, the prosthetic valve 10 can also include one or more skirts or sealing members. For example, the prosthetic valve 10 can include an inner skirt mounted on the inner surface of the frame 12. The inner skirt can function as a sealing member to prevent or decrease perivalvular leakage, to anchor the leaflets 26 to the frame 12, and/or to protect the leaflets against damage caused by contact with the frame during crimping and during working cycles of the prosthetic valve. The prosthetic valve 10 can also include an outer skirt mounted on the outer surface of the frame 12. The outer skirt can function as a sealing member for the prosthetic valve by sealing against the tissue of the native valve annulus and thus reducing paravalvular leakage around the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

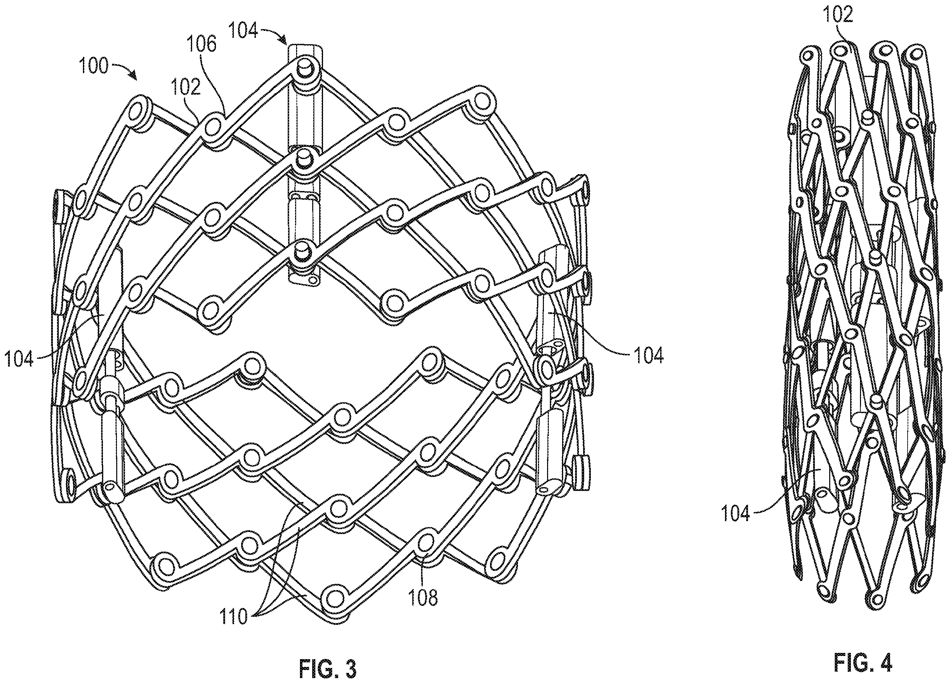

FIG. 3 shows a prosthetic heart valve 100, according to another embodiment. The prosthetic valve 100 can comprise three main components: an annular stent or frame 102, a valve structure (not shown), and one or more actuators 104 (e.g., three in the illustrated embodiment). The frame 102 can be configured to support the valve structure and to secure the prosthetic valve 100 within a native heart valve. The valve structure can be coupled to the frame 12 and/or to the actuators 104 and can be configured to allow blood to flow through the prosthetic valve 100 in one direction and to restrict blood flow in another direction. The actuators 104 can be coupled to the frame 102 and can be configured to adjust expansion of the frame 102 to a plurality of configurations including one or more functional or expanded configurations (e.g., FIG. 3), one or more delivery or compressed configurations (e.g., FIG. 4), and/or one or more intermediate configurations. The actuators 104 can also be configured to secure or lock the frame 102 in a desired configuration.

In this manner, the prosthetic valve 100 is generally similar to the prosthetic valve 10. One distinction between the prosthetic valve 100 and the prosthetic valve 10 is that the actuators 104 of the prosthetic valve 100 separate expansion/contraction actuation from locking actuation; whereas the actuators 16 of the prosthetic valve 10 combine expansion/contraction actuation and locking actuation. More specifically, the actuators 104 of the prosthetic valve 100 are configured such that axial (push/pull) actuation of components adjusts expansion of the frame 102 and separate rotational actuation of other components locks the frame in the desired configuration. Conversely, the actuators 16 of the prosthetic valve 10 are configured such rotational motion of components simultaneously adjusts and secures the frame 12 of the prosthetic valve 10. Configuring the actuators 104 with separate expansion and locking actuation can provide several advantages. Additional details and advantages of the actuators 104 of the prosthetic valve 100 are provided below.

The frame 102 of the prosthetic valve 100 comprises a first end portion 106 and a second end portion 108 and a plurality of struts 110. The struts 110 are interconnected and pivotably coupled together at junctions (e.g., via pins, rivets, etc.). In this manner, the struts 110 form joints allowing the frame 102 to be selectively adjusted to the expanded configuration (e.g., FIG. 3), the compressed configuration (e.g., FIG. 4), and one or more intermediate configurations between the expanded and compressed configurations.

Although the valve structure of the prosthetic valve 100 is not shown to better illustrate the actuators 104, the valve structure can comprises a plurality of leaflets. One or more portions of the leaflets (e.g., commissures) can be coupled to the frame 102 and/or the actuators 104. For example, in one embodiment the valve structure of the prosthetic valve 100 can be configured similar to the valve structure 14 of the prosthetic valve 10.

For simplicity, the description below primarily describes a single actuator 104 and its components, even though the prosthetic valve 100 comprises three actuators 104 distributed circumferentially around the frame 102. It should be understood that each actuator 104 can be configured and/or operate similarly. It should also be understood that in other embodiments the prosthetic valve 100 can comprise less than three actuators (e.g., 1-2) or more than three actuators (e.g., 4-6).

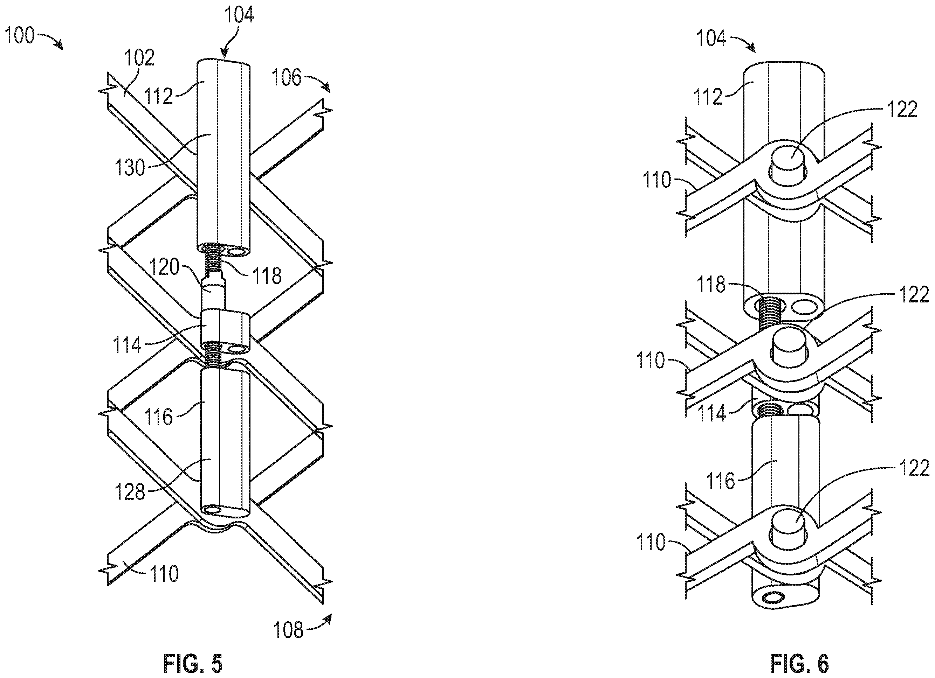

Referring now to FIG. 5, each actuator 104 of the prosthetic valve 100 can comprise a first or proximal support member 112, a second or intermediate support member 114, and a third or distal support member 116 (collectively or generically referred to as "the support members"). The proximal support member 112 can be coupled to the frame 102 at a first location (e.g., at or adjacent the first end portion 106 of the frame 102), the distal support member 116 can be coupled to the frame 102 at a second location (e.g., at or adjacent a second end portion 108 of the frame 102), and the intermediate support member 114 can be coupled to the frame 102 at a third location between the first and second locations.

Each actuator 104 can further comprise a locking member 118 and a locking nut 120. The locking member 118 can be fixedly coupled to the distal support member 116. The locking member 118 extends through and is movable (e.g., axially) relative to the intermediate support member 114 and the proximal support member 112. The locking nut 120 is disposed on the locking member 118 at a location between the proximal support member 112 and the intermediate support member 114. The locking nut 120 is movable relative to the locking member 118.

It should be noted that in some embodiments, the intermediate support member 114 can be omitted.

As shown in FIG. 6, the support members of the actuator 104 can be coupled to the frame 102 at joints of the struts 110. For example, in some embodiments, the support members can comprise projections 122 configured to extend through apertures of the struts 110. In other embodiments, the support members can be coupled to the frame at various other locations (e.g., between the joints) and/or with various other means for coupling (e.g., welding, adhesive, fasteners, etc.).

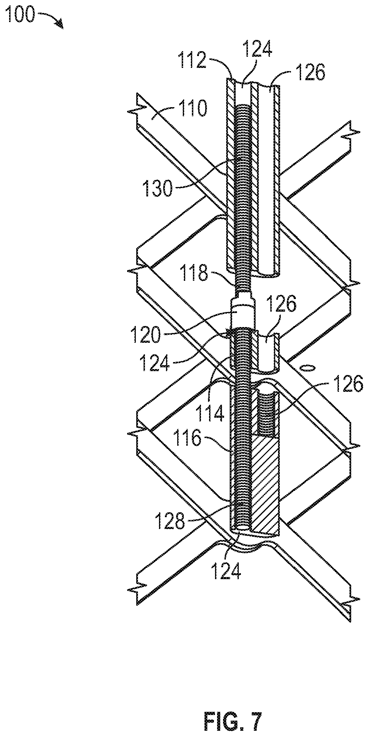

Referring to FIG. 7, each support member can comprise a locking lumen 124 and an actuation lumen 126 extending axially through at least a portion of the support member. The locking lumen 124 and the actuation lumen 126 of each support member can be spaced apart and parallel to each other. The locking lumens 124 of the actuator 104 extend coaxially through the support members and are configured to receive the locking member 118. The actuation lumens 126 of the actuator 104 extend coaxially through the support members and are configured to receive an actuation shaft of a delivery apparatus.

Referring still to FIG. 7, the locking member 118 of the actuator 104 can comprise a distal end portion 128 and a proximal end portion 130. The distal end portion 128 of the locking member 118 can be disposed within the locking lumen 124 of the distal support member 116. The locking member 118 can be coupled to the distal support member 116 in various ways (e.g., threads, fasteners, adhesive, welding, and/or other means for coupling. In other embodiments, the locking member 118 and the distal support member 116 can be integrally formed as a single, unitary component. The locking member 118 can extend proximally from the distal support member 116 and extend through the locking lumen 124 of the intermediate support member 114 toward the proximal support member 112.

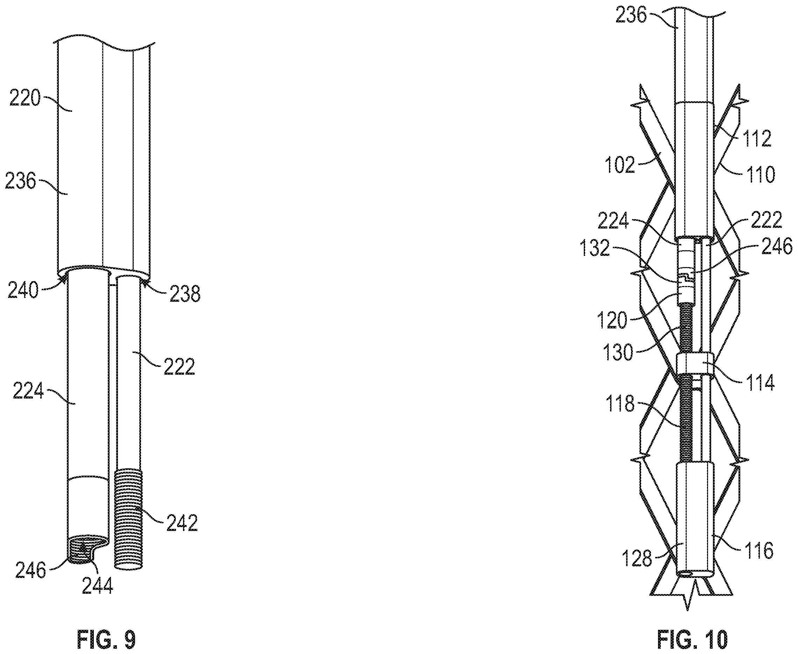

When the struts 110 of the frame 102 pivot relative to each other and the frame 102 moves between the expanded, compressed, and intermediate configurations, the proximal support member 112 and the intermediate support member 114 move axially relative to the locking member 118. This is because locking member 118 is fixed relative to the distal support member 116 and because the axial spacing between the support members changes as the frame 102 moves between the radially-expanded/axially-foreshortened configuration and the radially-compressed/axially-elongated configuration. For example, when the frame 102 is in a radially expanded configuration (e.g., FIG. 5), the axial spacing between the support members is relatively less, and thus the proximal end portion 130 of the locking member 118 can extend into the locking lumen 124 of the proximal support member 112. When the frame 102 is in a radially compressed configuration (e.g., FIG. 10), the axial spacing between the support members is relatively greater, and thus the proximal end portion 130 of the locking member 118 can be axially disposed between a distal end of the proximal support member 112 and a proximal end of the intermediate support member 114.

As mentioned above, the locking nut 120 can be disposed on and adjustably coupled to the locking member 118. For example, the locking member 118 can comprise external threads, and the locking nut 120 can comprise corresponding internal threads. In such embodiments, rotating the locking nut 120 in a first rotational direction (e.g., clockwise) relative to the locking member 118 moves the locking nut 120 in a first axial direction (e.g., distally) along the locking member 118, and rotating the locking nut 120 in a second rotational direction (e.g., counterclockwise) relative to the locking member 118 moves the locking nut 120 in a second axial direction (e.g., proximally) along the locking member 118.

The axial position of the locking nut 120 along the length of the locking member 118 limits the extent to which the intermediate support member 114 can move proximally relative to the locking member 118 and the distal support member 116. This is because the locking nut 120 is radially larger than and cannot pass through the locking lumen 124 of the intermediate support member 114. As such, the intermediate support member 114 can slide proximally along the locking member 118 until the locking nut 120 abuts the proximal end of the intermediate support member 114, as shown in FIGS. 5 and 7. Therefore, the frame 102 of the prosthetic valve 100 can be radially compressed to a greater extent when the locking nut 120 is disposed toward the proximal end portion 130 of the locking member 118 than when the locking nut 120 is disposed toward the distal end portion 128 of the locking member 118. In this manner, the locking nut 120 can act as a proximal stop for the intermediate support member 114 along the locking member 118 and as a locking mechanism to prevent the frame 102 from being radially compressed beyond a desired extent.

The intermediate support member 114 and/or the distal support member 116 can limit the extent to which the frame 102 of the prosthetic valve 100 can be radially expanded. This is because the frame 102 of the prosthetic valve 100 can expand radially outwardly until the intermediate support member 114 and the distal support member 116 contact each other, which restricts further radial expansion of the frame 102 of the prosthetic valve 100. More specifically, the intermediate support member 114 can translate distally over the locking member 118 until the distal end of the intermediate support member 114 abuts the proximal end of the distal support member 116.

In this manner, the distal support member 116 can act as a distal stop and/or a locking mechanism for the intermediate support member 114 along the locking member 118, which prevents the frame 102 from being radially expanded beyond a desired extent. As such, the axial length of the intermediate support member 114 and/or the distal support member 116 can be selected to determine the extent to which the frame 102 of the prosthetic valve 100 can be radially expanded. For example, the frame 102 of the prosthetic valve 100 can expand radially outwardly to a lesser extent when the intermediate support member 114 and/or the distal support member 116 are relatively longer because the intermediate support member 114 has less distance to travel along the locking member 118 before the distal end of the intermediate support member 114 abuts the proximal end of the distal support member 116 than when the intermediate support member 114 and/or the distal support member 116 are relatively shorter.

As mentioned above, the actuation lumens 126 of the actuators 104 can be configured to receive an actuation shaft of a delivery apparatus such that the actuation shaft of the delivery apparatus can extend through the proximal support member 112, extend through the intermediate support member 114, and be selectively coupled to the distal support member 116. Additional details regarding the actuators 104 of the prosthetic valve 100 and the actuation shaft of the delivery apparatus are provided below. The actuation shaft can, for example, be used to move the distal support member axially relative to the intermediate support member 114 and the proximal support member 112, and thereby move the prosthetic valve 100 between the compressed configuration and the expanded configuration.

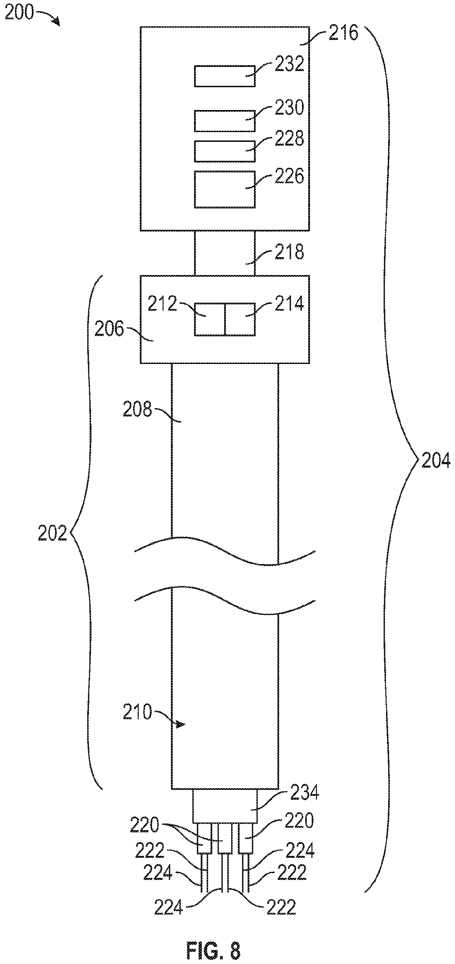

FIG. 8 shows a schematic representation of a delivery apparatus 200. The delivery apparatus 200 can be used, for example, to deliver a prosthetic valve to an implantation location within a patient and to manipulate the prosthetic valve within the patient's body. Exemplary manipulations include: positioning the prosthetic valve relative to an implantation location, radially expanding and compressing the prosthetic valve, securing the prosthetic valve at the implantation location, releasing the prosthetic valve from the delivery apparatus 200, etc.

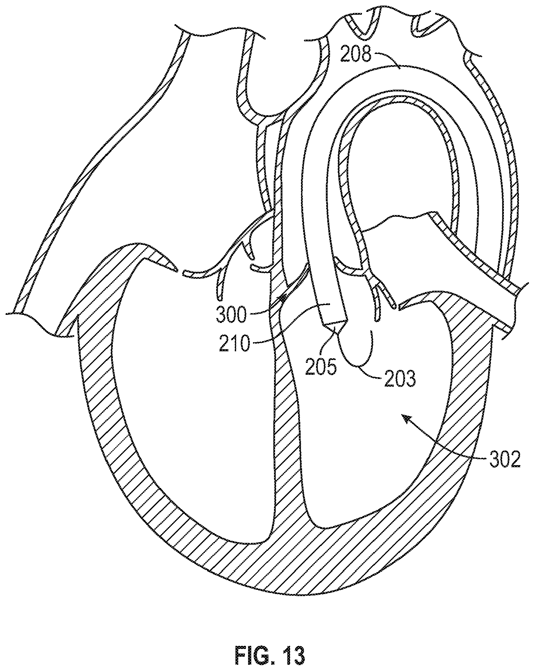

Referring still to FIG. 8, the delivery apparatus 200 can comprise one or more catheters. For example, in the illustrated embodiment, the delivery apparatus 200 includes a first or outer catheter 202 and a second or valve catheter 204. The valve catheter 204 can extend axially through the outer catheter 202. Generally speaking, the outer catheter 202 can be configured to receive and retain a prosthetic valve in a radially compressed configuration while the prosthetic valve is inserted into a patient's body and advanced to an implantation location, and the valve catheter 204 can be configured to have a prosthetic valve can be coupled to the distal end portion of the valve catheter and to manipulate the prosthetic valve at the implantation location. In some embodiments, the delivery apparatus 200 can include one or more other components (e.g., a guide wire 203, a nose cone 205, etc. (see FIG. 13)) and/or one or more additional catheters (e.g., a introducer). In other embodiments, the delivery apparatus can omit one or more components (e.g., the outer catheter 202).

The outer catheter 202 of the delivery apparatus 200 can include a first handle portion 206 and a first shaft 208. The handle portion 206 can be disposed at a proximal end of the outer catheter 202 can be configured to be disposed outside of a patient's body during an implantation procedure. The first shaft 208 can extend distally from the handle portion 206 and can include one or more lumens (not shown), at least one of which is configured such that the valve catheter 204 can extend therethrough. A distal end portion of the first shaft 208 can comprise a sheath 210 configured to receive and retain a prosthetic valve in a radially compressed configuration while the prosthetic valve is inserted into a patient's body and advanced to an implantation location. The sheath 210 can, for example, protect the prosthetic valve and/or the patient's native anatomy (e.g., the patient's femoral artery and/or aorta) while the prosthetic valve as the prosthetic valve is advanced to the implantation location (e.g., the patient's native aortic valve).

In some embodiments, the outer catheter 202 can include a positioning mechanism 212 configured to move the outer catheter 202 relative to the valve catheter 204. The positioning mechanism 212 can be used, for example, to deploy a prosthetic valve from the sheath 210 of the outer catheter 202. In certain instances, the positioning mechanism 212 can be disposed within the handle portion 206 of the outer catheter 202. Additional details regarding positioning mechanisms can be found, for example, in U.S. Patent Nos. 8,652,202, 9,155,619, 9,867,700, and 10,588,744.

The outer catheter 202 can, in some embodiments, include a steering mechanism 214 configured such that a user can from a location outside the patient's body position the distal end portion of the first shaft 208 relative to a patient's native anatomy. In certain instances, the steering mechanism 214 can be disposed within the handle portion 206 of the outer catheter 202. Additional details regarding the outer catheter and steering mechanisms can be found, for example, in U.S. Patent Nos. 10,076,638 and 10,653,862.

The valve catheter 204 of the delivery apparatus 200 can comprise a second handle portion 216, a second shaft 218, one or more third shafts 220, one or more actuation shafts 222, and one or more locking shafts 224. For example, there are three of each of the shafts 220, 222, 224 in the illustrated embodiment. In other embodiments, there can be fewer than three (e.g., 1-2) or more than three (e.g., 4-6) of each of the shafts 220, 222, 224.

Proximal end portions of the shafts 218, 220, 222, 224 can be coupled to and/or housed within the second handle portion 216. The shafts 218, 220, 222, 224 can extend distally from the second handle portion 216, and the shafts 218, 220, 222, 224 can be movable (e.g., axially and/or rotationally) relative to the second handle portion 216 and relative to each other. The second handle portion 216 can be configured to move the valve catheter 204 relative to the outer catheter 202 and/or to move the shafts 218, 220, 222, 224 relative to each other and/or other components of the delivery apparatus. The second shaft 218 can be configured to house the third shafts 220. The third shafts 220 can extend axially through the second shaft 218 and can be configured to house at least a portion of the shafts 222, 224 and to engage actuators of a prosthetic valve. The actuation shafts 222 can extend axially through the third shafts 220 and can be configured to actuate the actuators of the prosthetic valve. The locking shafts 224 can extend axially through the third shafts 220 and can be configured to actuate and/or lock the actuators of the prosthetic valve. Additional details of these components and their interaction together and/or with a prosthetic valve are described below.

In some embodiments, the second handle portion 216 can comprise a second positioning mechanism 226, a second steering mechanism 228, an actuation mechanism 230, and/or a locking mechanism 232. The second positioning mechanism 226 can be configured to move the third shafts 220 relative to the second shaft 218. The second steering mechanism 228 can be configured to steer the distal end portion of the second shaft 218 (and thus the shafts 220, 222, 224). The actuation mechanism 230 can be configured to move the actuation shafts 222 relative to the third shafts 220 (e.g., either collectively or individually). The locking mechanism 232 can be configured to move the locking shafts 224 relative to the third shafts 220 (e.g., either collectively or individually). The mechanisms of the delivery apparatus 200 can comprise mechanical components (e.g., knobs, handles, buttons, levers, gears, drive screws, nuts, pull wires etc.) and/or electrical components (e.g., motors, circuitry, wires, etc.). These mechanisms can, for example, make positioning and/or moving the shafts relative to each other relatively easier than manual manipulation. These mechanisms can also allow one or more of the movements to be automated.

In other embodiments, one or more of the mechanisms 226, 228, 230, 232 can be omitted from the second handle portion 216, and the shafts 218, 220, 222, 224 can be positioned and/or moved (e.g., pushed/pulled, rotated, etc.) relative to each other manually. Configuring the second handle portion 216 in this manner can, for example, reduce the number of components and/or make the device simpler and/or more cost effective to manufacture.

The second shaft 218 of the valve catheter 204 can be configured to extend distally from the second handle portion 216 of the valve catheter 204 and through first handle portion 206 and the first shaft 208 of the outer catheter 202 such that a distal end portion 234 of the second shaft extends distally beyond the distal end portion of first shaft 208. This can allow a prosthetic valve (which can be coupled to the distal end portion of the valve catheter 204) to be deployed from the sheath 210 of the outer catheter 202 by moving the second shaft 218 (and thus the shafts 220, 222, 224) relative to the first shaft 208.

The second shaft 218 can comprise one or more (e.g., three) lumens. Each lumen can be configured to receive a respective third shaft 220.

Each of the third shafts 220 can be configured to extend distally from the second handle portion 216 through a respective lumen of the second shaft 218 such that distal end portions of the third shafts 220 can be disposed distally relative to the distal end portion of the second shaft 218. In this manner, the distal end portions 236 of the third shafts 220 can contact proximal support members of the actuators of the prosthetic valve, as shown in FIG. 10. As such, the third shafts 220 can, for example, be used to apply a distally directed force on the proximal support members 112 (e.g., while the actuation shafts 222 apply a proximally directed force on the distal support members 116).

As shown in FIG. 9, each of the third shafts 220 can comprise a plurality of lumens, including an actuation lumen 238 and a locking lumen 240. The actuation lumen 238 of each third shaft 220 can be configured to receive a respective actuation shaft 222. The locking lumen 240 of each third shaft 220 can be configured to receive a respective locking shaft 224.

Referring still to FIG. 9, each actuation shaft 222 can be configured to extend distally from the second handle portion 216 through an actuation lumen 238 of a respective third shaft 220 such that the distal end portion of the actuation shaft 222 can be disposed distally relative to the distal end portion 236 of the third shaft 220. As such, the prosthetic valve 100 can be releasably coupled to the distal end portions of the actuation shafts 222.

The distal end portion of each actuation shaft 222 can comprise a coupling element configured to releasably couple the actuation shaft 222 to a respective actuator of a prosthetic valve. The coupling element can be coupled to or integrally formed with the actuation shaft 222. For example, in the illustrated embodiment, the coupling element of the actuation shaft 222 comprise a threaded element 242 disposed at the distal end portion of the actuation shaft 222. The threaded element 242 comprises external threads configured to mate with corresponding internal threads disposed within the actuation lumen 126 of the distal support member 116 of the prosthetic valve 100, as shown in FIG. 10. When the threaded element 242 is coupled to the distal support member 116 of the prosthetic valve 100, the actuation shaft 222 can be used to actuate the actuators 104 of the prosthetic valve 100 and thus move the prosthetic valve 100 between the radially expanded and radially compressed configurations, as further explained below.

Referring again to FIG. 9, each locking shaft 224 can be configured to extend distally from the second handle portion 216 through a locking lumen 240 of a respective third shaft 220 such that the distal end portion of the locking shaft 224 can be disposed distally relative to the distal end portion 236 of the third shaft 220. As such, the prosthetic valve 100 can be releasably coupled to the distal end portions of locking shafts 224.

The distal end portion of each locking shaft 224 can comprise a lumen or bore 244 configured to receive the locking member 118 of the prosthetic valve 100. In some embodiments, the bore 244 of locking shaft 224 can comprise internal threads configured to mate with corresponding external threads of the locking member 118 of the prosthetic valve 100 such that the locking shaft 224 can be releasable coupled to the locking member 118. As a result, rotating the locking shaft 224 of the delivery apparatus 200 relative to the locking member 118 of the prosthetic valve 100 causes the locking shaft 224 to translate proximally or distally along the locking member 118 depending on the direction in which the locking shaft 224 is rotated.

The distal end portion of each locking shaft 224 can comprise a mating element (e.g., a projection 246) configured to mate with a corresponding mating element (e.g. a projection 132) of the locking nut 120 of the prosthetic valve 100. The mating elements of the locking shaft 224 and the locking nut 120 can allow the locking shaft 224 and the locking nut 120 to selectively mate together. The mating elements can be configured such when the locking shaft 224 and the locking nut 120 are mated together by the mating elements, rotating the locking shaft 224 in a first direction (e.g., clockwise) relative to the locking member 118 results in the locking nut 120 rotating together with the locking shaft 224 in the first direction relative to the locking member 118. The mating elements can also be configured such when the locking shaft 224 and the locking nut 120 are mated together by the mating elements, rotating the locking shaft 224 in a second direction (e.g., counterclockwise) relative to the locking member 118 results in the locking shaft 224 rotating relative to the locking nut 120 and the locking nut 120 maintaining its position relative to the locking member 118. In other words, the mating features can be configured such that locking shaft 224 can be used to move the locking nut 120 in a first axial direction (e.g., distally) but not in a second axial direction (e.g., proximally). In this manner, the locking shaft 224 together with the locking nut 120 can be used to lock the frame 102 of the prosthetic valve 100 in a desired radially expanded configuration, as further described below.

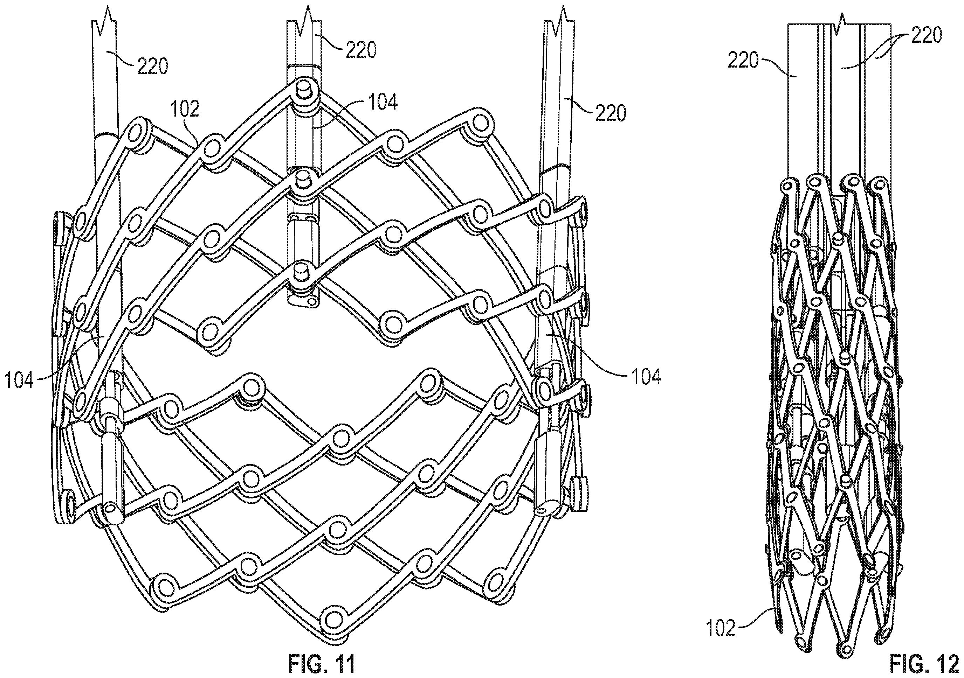

The prosthetic valve 100 can be releasably coupled to the distal end portion of the delivery apparatus 200 to form a delivery assembly, as shown in FIG. 11. This can be accomplished, for example, by positioning the first end portion 106 of the prosthetic valve 100 adjacent to the distal end portion of the delivery apparatus 200 such that the distal end portions 236 of the thirds shafts 220 are aligned with the proximal support members 112 of the prosthetic valve 100. The actuation shafts 222 of the delivery apparatus 200 can then be advanced distally through respective actuation lumens 126 of the prosthetic valve 100 until the threaded elements 242 of the actuation shaft 222 contact the actuation lumens 126 of respective distal support members 116. The actuation shafts 222 can then be releasably coupled to the distal support members 116 by rotating the actuation shafts 222 in a first direction (e.g., clockwise) relative to the distal support members 116 such that the threaded elements 242 of the actuation shafts 222 mate with the distal support members 116. The locking shafts 224 of the delivery apparatus 200 can then be advanced distally through respective locking lumens 124 of the prosthetic valve 100 until the distal end portions of the locking shafts 224 contact respective locking members 118 of the prosthetic valve 100. The locking shafts 224 can then be releasably coupled to the locking member 118 by rotating the locking shafts 224 in a first direction (e.g., clockwise) relative to the locking members 118 such that the internal threads of the locking shafts 224 threadably mate with external threads of respective locking members 118 and proximal ends of the locking members 118 are disposed within respective bores 244 of the locking shafts 224.

The locking nut 120 of the prosthetic valve 100 can be disposed at or adjacent the proximal end portion 130 of the locking member 118. This can allow the intermediate support member 114 of the prosthetic valve 100 to slide proximally relative to the locking member 118, which allows the prosthetic valve 100 to be moved to the radially compressed configuration. With the locking nut 120 disposed at or adjacent the proximal end portion 130 of the locking member, the intermediate support member 114 of the prosthetic valve 100 can also slide distally relative to the locking member 118, which allows the prosthetic valve to be moved to the radially expanded configuration.

With the prosthetic valve 100 coupled to the delivery apparatus 200, the prosthetic valve 100 can be moved to the radially compressed configuration. This can be accomplished moving the actuation shafts 222 distally relative to the third shafts 220, which applies an axially tensile force to the frame 102 of the prosthetic valve 100. Additionally or alternatively, this can be accomplished by applying a radially compressive force to the frame 102 of the prosthetic valve (e.g., with a crimping device). This causes the frame 102 of the prosthetic valve 100 to axially elongate and radially compress, as shown in FIG. 12.

The radially compressed prosthetic valve 100 can then be loaded into the sheath 210 of the delivery apparatus 200 (see FIG. 13) (or advanced into a patient's vasculature in a sheath-less configuration). This can be accomplished by moving the outer catheter 202 distally relative to the valve catheter 204, and/or by moving the valve catheter proximally relative to the outer catheter 202.

The delivery assembly can then be inserted into a patient's body, and the prosthetic valve 100 can be advanced to an implantation location. A delivery assembly can be configured for various implantation locations (e.g., a native aortic valve, mitral valve, tricuspid valve, and/or pulmonary valve) and/or delivery approaches (e.g., transfemoral, transapical, transseptal, etc.). For example, in the illustrated embodiment, the delivery assembly is configured for implanting the prosthetic valve 100 within a native aortic valve using a transfemoral approach.

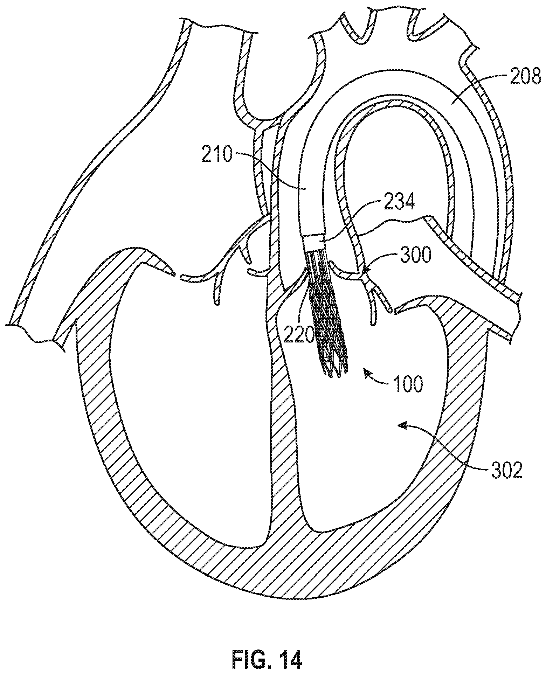

FIG. 13 shows the distal end portion of the delivery apparatus 200 inserted through a patient's native aortic annulus 300 such that the nose cone 205 and the sheath 210 are disposed within the patient's left ventricle 302. The prosthetic valve 100 can then be deployed from the sheath 210 of the delivery apparatus 200, as shown in FIG. 14. This can be accomplished, for example, by moving the first shaft 208 of the outer catheter 202 proximally relative to the valve catheter 204 (e.g., via the positioning mechanism 212). To better illustrate the prosthetic valve 100, the guide wire 203 and the nose cone 205 are not shown in FIGS. 14-16.

With the prosthetic valve 100 exposed from the sheath 210 of the delivery apparatus 200, the delivery apparatus 200 can be used to position the prosthetic valve 100 within the native annulus and to radially expand the prosthetic valve 100. Positioning the prosthetic valve 100 can include axial and/or rotational movement of the first and second shaft 208, 218 and/or actuating one or more of the first and second steering mechanisms 214, 228.

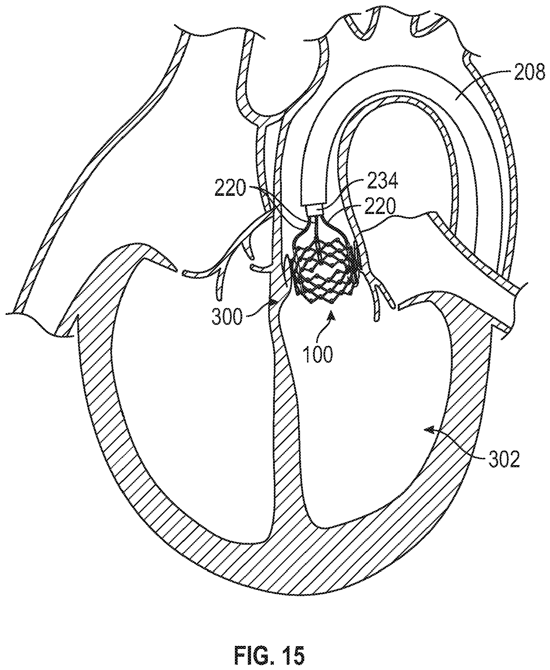

Referring to FIG. 10, the prosthetic valve 100 can be radially expanded by moving the actuation shafts 222 of the delivery apparatus 200 proximally relative to the third shafts 220 of the delivery apparatus 200 (e.g., by actuating the actuation mechanism 230) and/or by moving the third shafts 220 distally relative to the actuation shafts 222. This causes the delivery apparatus 200 to apply an axially compressive force on the actuators 104 the frame 102 of the prosthetic valve 100. Specifically, the actuation shafts 222 apply a proximally directed force to the distal support members 116 of the prosthetic valve 100 and the third shafts 220 apply an opposing distally directed force to the proximal support members 112 of the prosthetic valve 100. As a result, the struts 110 of frame 102 pivot relative to each other and the prosthetic valve 100 radially expands, as shown in FIG. 15.

A user can select the extent to which the prosthetic valve 100 is radially expanded. For example, the user can radially expand the prosthetic valve 100 to a predetermined radial expansion. The user can then determine whether paravalvular leakage exists. If the user determines adjustments (e.g., in positioning and/or expansion) need to be made, the user can further radially expand the prosthetic valve 100 by moving the actuation shafts 222 proximally relative to the third shafts 220. Alternatively, the user can radially compress the prosthetic valve 100 by moving the actuation shafts 222 distally relative to the third shafts 220, or vice versa. This reduces axial compression on the frame 102 of the prosthetic valve 100, which allows the frame 102 to axially elongate and radially compress. The prosthetic valve 100 can be repositioned relative to the native aortic annulus 300. The prosthetic valve 100 can also be further compressed and retrieved into the sheath 210 of the delivery apparatus 200.

Once the prosthetic valve 100 is desirably positioned and radially expanded relative to the native aortic annulus 300, the prosthetic valve 100 can be locked in the desired radially expanded configuration. This can be accomplished by actuating the locking mechanism 232 of the delivery apparatus 200 such that the locking shafts 224 rotate in a first direction and move distally relative to the locking members 118 of the prosthetic valve 100. Referring again to FIG. 10, when the locking shafts 224 engage the locking nuts 120, the locking nuts 120 of the prosthetic valve 100 also rotate in the first direction and move distally relative to the locking members 118. The locking nuts 120 can be moved distally relative to the locking members 118 until the locking nuts 120 contact the intermediate support members 114 (see, e.g., FIG. 5). Thus, the locking nuts 120 restrict intermediate support members 114 from moving proximally relative to the locking members 118, and thereby restrict the frame 102 of the prosthetic valve 100 from being radially compressed.

Since the actuation shafts 222 bear the load for expanding the prosthetic valve 100, the locking shafts 224 can be rotated relatively easily relative to the locking members 118. This can, for example, reduce the likelihood that the locking shafts 224, the locking nut 120, and/or the locking members 118 will bind and/or become damaged (e.g., stripped threads).

With the prosthetic valve 100 secured within the native aortic annulus and the radial expansion of the prosthetic valve 100 locked, the prosthetic valve 100 can be released from the delivery apparatus 200, and the delivery apparatus 200 can be removed from the patient. The locking shafts 224 of the delivery apparatus 200 can be released from the prosthetic valve 100 by actuating the locking mechanism 232 of the delivery apparatus 200 such that the locking shafts 224 rotate in a second direction and move proximally relative to the locking nuts 120 and the locking members 118 of the prosthetic valve 100 until the locking shafts 224 are uncoupled from the proximal end portions 130 of the locking members 118 and the locking members 118 are withdrawn from the bores 244 of the locking shafts 224. The actuation shafts 222 of the delivery apparatus 200 can be released from the prosthetic valve 100 by rotating the actuation shafts 222 in a second direction relative to the distal support members 116 of the prosthetic valve 100 until the actuations shafts 222 are withdrawn from the locking lumens 126 of the distal support members 116.

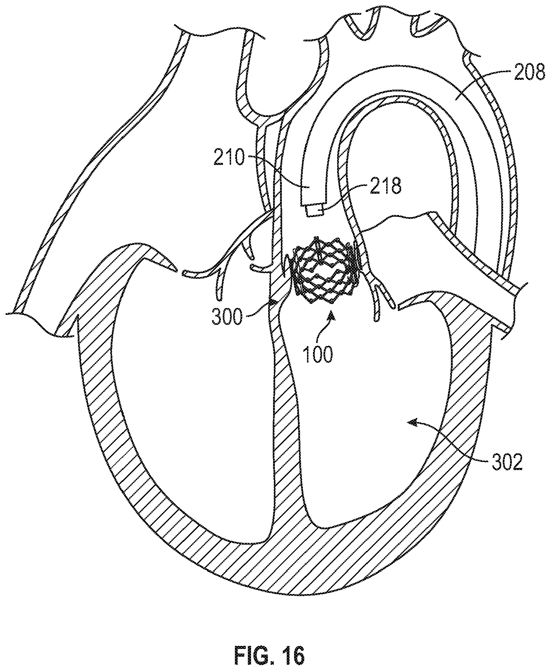

With the prosthetic valve 100 released from the delivery apparatus 200, the third shafts 220 of the delivery apparatus 200, together with the actuation shafts 222 and the locking shafts 224, can be moved proximally relative to the second shaft 218 such that the shafts 220, 222, 224 are disposed within the second shaft 218 (see FIG. 16). The first and second catheters 202, 204 can be withdrawn from the patient's body.

The delivery assembly comprising the prosthetic valve 100 and the delivery apparatus 200 can provide one or more advantages over prior mechanically expandable prosthetic valves. For example, the delivery assembly separates the actuation mechanism from the locking mechanism. This can reduce the radial profile of the actuators 104 of the prosthetic valve 100 because the actuation and locking components are adjacent rather than coaxial, which in turn can reduce the radial profile of the prosthetic valve in the compressed configuration. Yet another advantage of this configuration is that there is little or no load on the locking shafts 224 and locking nuts 120 when they are being rotated because the actuation shafts 222 bear the load (e.g., tension) need to expand the prosthetic valve 100, which can reduce binding and increase reliability of the assembly. Also, the locking shafts 224 can be solid (with the exception of the bores 244 at the distal end portions), which can improve strength of the shafts against forces that may damage the shafts.

The separation of the actuation mechanism from the locking mechanism can also increase redundancy and thus safety of the prosthetic valve by providing multiple actuation mechanisms and multiple locking mechanisms. For example, if one of the locking mechanisms of the prosthetic valve 100 were to fail, there are other locking mechanisms that would prevent the prosthetic valve from radially compressing. Also, in lieu of or addition to the actuation shafts, the prosthetic valve 100 can be expanded by rotating the locking nut 120 via the locking shafts 224 to expand/compress the prosthetic valve 100. In this manner, the locking nut 120 and the locking member 118 act similar to the rod 30 of the prosthetic valve 10.

This configuration can allow the prosthetic valve 100 to be expanded relative quickly via the axial (which can also be referred to as "linear") actuation mechanism, while also ensuring that the prosthetic valve 100 is secured in the expanded configuration via the rotational locking mechanism.

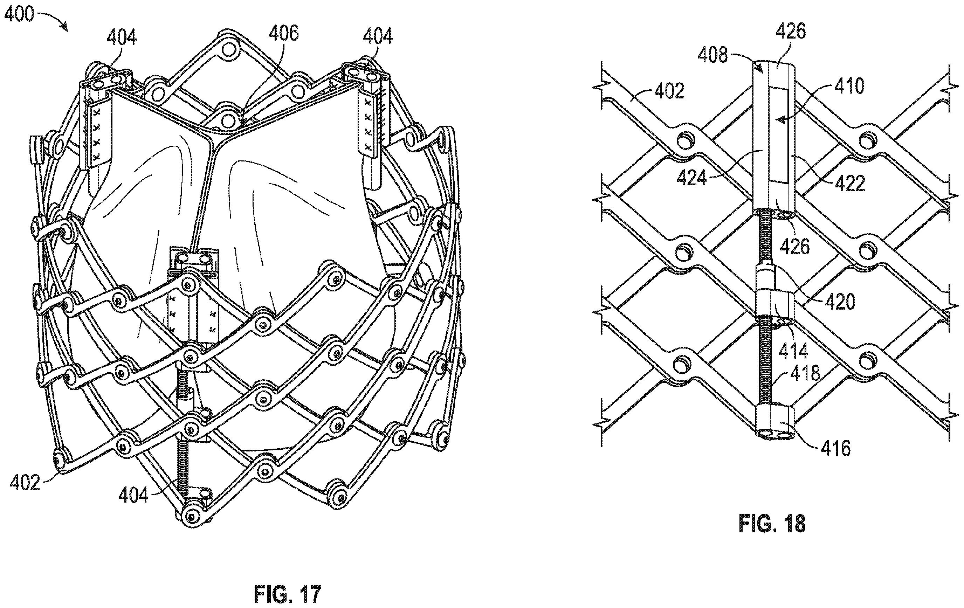

FIG. 17 shows a prosthetic heart valve 400, according to another embodiment. The prosthetic valve 400 is similar to the prosthetic valve 100 in that it comprises a frame 402, one or more actuators 404 for expanding/compressing the frame 402, and a valve structure 406 configured for allowing blood flow through the frame 402 in one direction. One difference between the prosthetic valve 400 and the prosthetic valve 100 is that proximal support members 408 of the actuators 404 comprise windows 410 configured to receive leaflet commissures 412 of the valve structure 406, as shown in FIGS. 18-19. Additional details regarding these components are provided below.

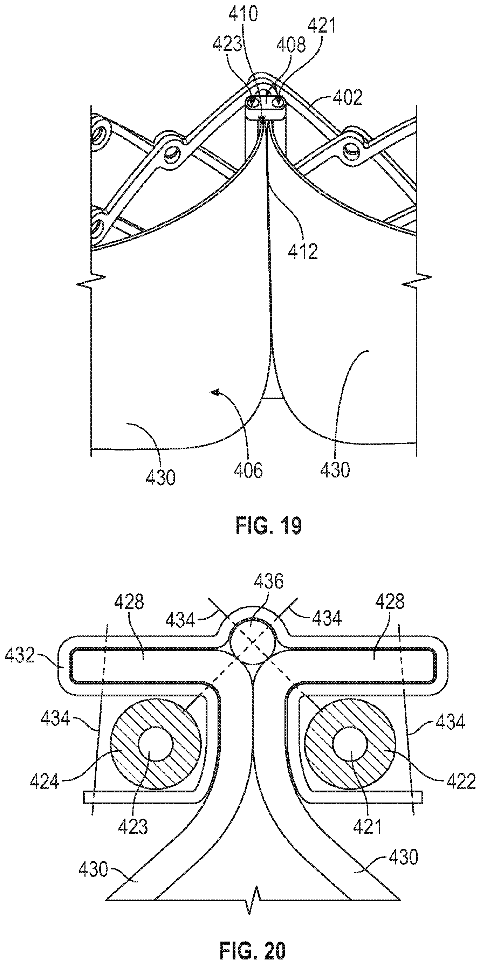

Referring to FIG. 18, the actuators 404 of the prosthetic valve 400 can each comprise a proximal support member 408, an intermediate support member 414, a distal support member 416, a locking member 418, and a locking nut 420. As shown in FIG. 19, the support members 408, 414, 416 can each have an actuation lumen 421 and a locking lumen 423. The actuation lumens 421 can be configured for receiving an actuation shaft of a delivery apparatus. The locking lumens 423 can be configured for receiving the locking member 418 and a locking shaft of a delivery apparatus.

The intermediate support member 414, the distal support member 416, the locking member 418, and the locking nut 420 of the prosthetic valve 400 can be configured similar to the intermediate support member 114, the distal support member 116, the locking member 118, and the locking nut 120 of the prosthetic valve 100, respectively.

Referring again to FIG. 18, the proximal support member 408 of each actuator 404 can comprise an actuation tube 422, a locking tube 424, and one or more connection portions 426 (e.g., two in the illustrated embodiment). The actuation lumen 421 can extend axially through the actuation tube 422, and the locking lumen 423 can extend axially through the locking tube 424. The tubes 422, 424 can be spaced apart from each other, and the connection portions 426 can be disposed at the proximal and/or distal end portions of the tubes 422, 424 and extend therebetween. In this manner, the tubes 422, 424 and the connection portions 426 define the window 410. As shown in FIG. 19, the window 410 can be configured to receive the commissures 412 of the valve structure 406. The connection portions 426 can also be configured for mounting the proximal support member 408 to the frame 402.

Referring now to FIG. 20, the commissures 412 can be formed by tabs 428 of adjacent pairs of the leaflets 430. The tabs 428 of a pair of leaflets 430 can extend radially through the window 410 of the proximal support member 408. The tabs 428 can then flare circumferentially away from each other and extend at least partially around a respective tube 422, 424 of the proximal support member 408.

In some embodiments, a coupling member 432 (e.g., a fabric strip) configured for securing the tabs 428 of the leaflets 430 to the tubes 422, 424 and/or protecting the leaflets 430 from the frame 402 and actuators 404 can be provided. The coupling member 432 can extend between the leaflets 430 and the tubes 422, 424. The coupling member 432 and the tabs 428 can be coupled together in various ways (e.g., via sutures 434, adhesive, and/or other coupling means).

In some embodiments, the prosthetic valve 400 can comprise a wedge element 436. The wedge element 436 can be disposed radially between the commissures 412 of the leaflets 430 and the frame 402. The wedge element 436 can, for example, help to the secure the tabs 428 of the leaflets 430 together (e.g., via the sutures 434) and/or reduce abrasion between the leaflets 430 and the frame 402.

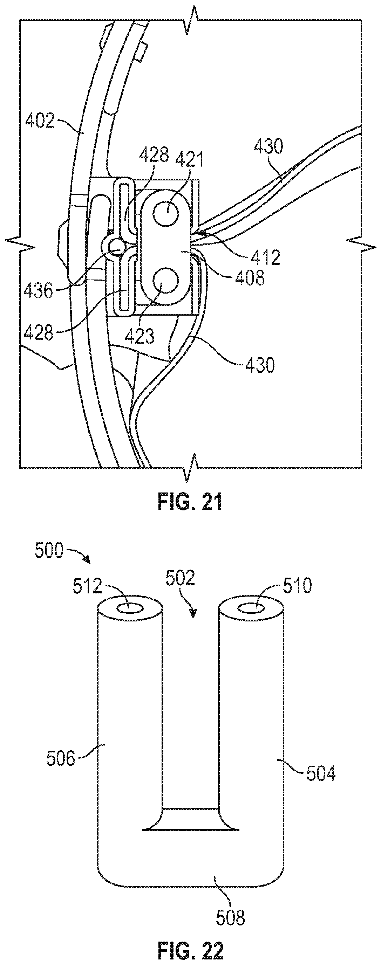

Attaching the commissures 412 of the leaflets 430 to the proximal support members 408 of the actuators 404 in this manner can provide several advantages. For example, this configuration can provide a radial space between the commissures 412 and the frame 402, as shown in FIG. 21. The radial space can reduce the likelihood that the leaflets 430 will rub against the frame 402, which can prolong the longevity of the prosthetic valve 400. The radial space can also prevent smooth muscle cell proliferation from the native tissue towards the commissures 412, which can improve the functionality and/or longevity of the prosthetic valve 400.

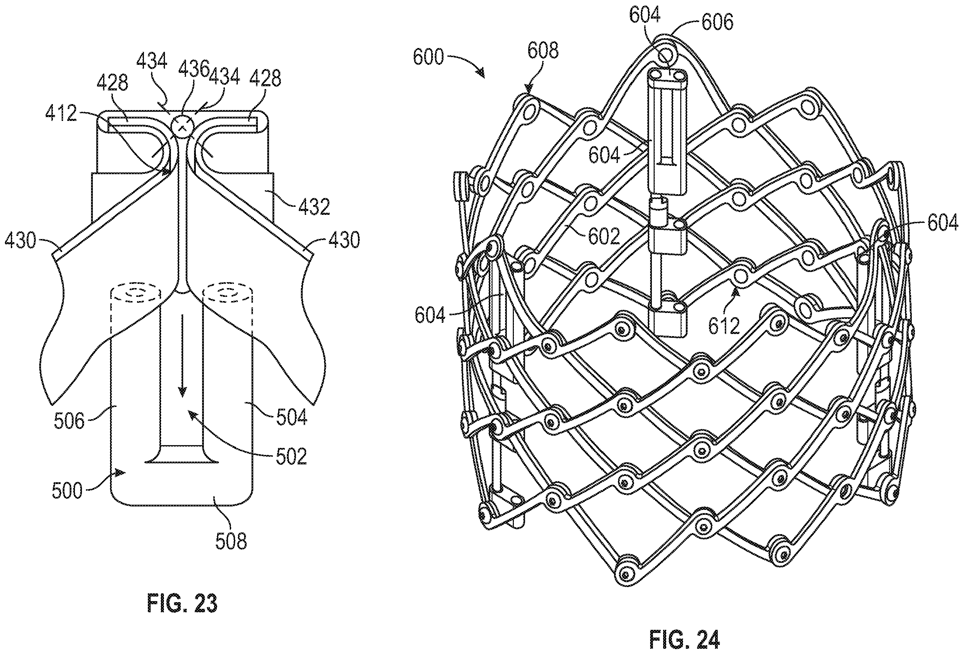

FIG. 22 shows a proximal support member 500, according to another embodiment. The proximal support member 500 can be used, for example, in lieu of the proximal support member 408 of the prosthetic valve 400. The proximal support member 500 is configured generally similar to the proximal support member 408, but the proximal support member 500 has an open slot 502 rather than the enclosed window 410 of the proximal support member 408. As shown in FIG. 23, forming the proximal support member 500 in this manner can allow a valve structure (e.g., the valve structure 406) to be at least partially preassembled, and then the commissure 412 can be inserted into the open slot 502 of the proximal support member 500. This can, for example, make assembling a prosthetic valve easier, improve manufacturing throughput, and/or improve quality.

The proximal support member 500 can comprise an actuation tube 504, a locking tube 506, and a connection portion 508. The tubes 504, 506 can be spaced apart from each other and the connection portion 508 can extend between distal end portions of the tubes 504, 506. The actuation tube 504 can comprise an actuation lumen 510 configured to receive an actuation shaft of a delivery apparatus (e.g., the actuation shaft 222). The locking tube 506 can comprise a locking lumen 512 configured to receive a locking shaft of a delivery apparatus (e.g., the locking shaft 224). The connection portion 508 can be configured for mounting the proximal support member 500 to a frame of a prosthetic valve (e.g., the frame 402).

The slot 502 of the proximal support member 500 is defined by the tubes 504, 506, and the connection portion 508. In the illustrated embodiment, the slot 502 is generally "U" shaped. In other embodiments, the slot 502 can be tapered or "V" shaped or comprise another shape configured for receiving the commissure of a valve structure.

As shown in FIG. 23, the commissure 412 of the valve structure 406 can be inserted into the slot 502 of the proximal support member 500. The valve structure can have the tabs 428 of the leaflets 430 coupled together via the coupling member 432, the wedge 436, and the sutures 434. With the commissure 412 disposed in the slot 502, the commissure 412 can be secured to the proximal support member 500 by wrapping the coupling member 432 around the tubes 504, 506 of the proximal support member 500 and suturing the coupling member 432 to the tubes 504, 506 similar to the manner shown in FIG. 20. In addition or alternatively, the commissures 412 can be secured to the proximal support member 500 with another means for coupling, such as adhesive, fasteners, etc.

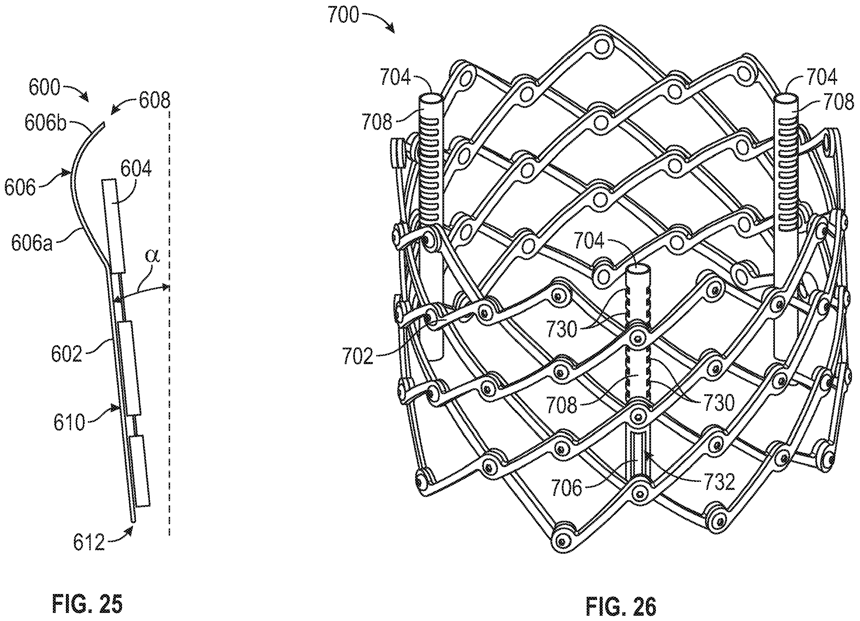

FIG. 24 shows a prosthetic heart valve 600, according to another embodiment. The prosthetic valve 600 can comprise a frame 602 and one or more actuators 604. Although not shown, the prosthetic valve 600 can also comprise a valve structure and/or one or more sealing skirts. The prosthetic valve 600 is configured generally similar to the prosthetic valve 400 except that the frame 602 of the prosthetic valve 600 comprise one or more flared apices 606 at an outflow end 608 of the frame and adjacent to the actuators 604, as best shown in FIG. 25.

Referring to FIG. 25, each flared apex 606 of the frame 602 can comprise a first portion or segment 606a and a second portion or segment 606b. The first segment 606a can extend radially outwardly relative to the main body 610 of the frame 602 and relative to the actuator 604 so as to create a space or a gap between the frame 602 and the actuator 604 (and the commissure, which can be coupled to the actuator). The second segment 606b can extend radially inwardly from the first segment 606b and radially overlap (at least partially) with the actuator 604. The second segment 606b can help to reduce contact between the apices of the frame 602 and the native tissue, which can reduce trauma to the native tissue (e.g., at the sinotubular junctions).

In some embodiments, the main body 610 of the frame 602 can taper radially outwardly from the inflow end 612 to the outflow end 608. The degree of taper or draft angle α can be measured relative to a vertical line. The draft angle α can vary. For example, in some embodiments, the draft angle α can be within a range of 2-15 degrees. In certain embodiments, the draft angle α can be within a range of 5-10 degrees.

As shown in FIG. 24, the frame 602 of the prosthetic valve 600 has three flared apices 606 which are circumferentially aligned with the actuators 604. In other embodiments, the frame can include less or more than three flared apices. For example, in some embodiments, each apex of the outflow end of the frame can be flared. As another example, the prosthetic valve can have two actuators, and the frame can comprise two flared apices circumferentially aligned with the actuators.

FIG. 26 shows a prosthetic heart valve 700, according to yet another embodiment. The prosthetic valve 700 can comprise a frame 702 and one or more actuators 704 (e.g., three in the illustrated embodiment). The frame 702 of the prosthetic valve 700 can be configured to support a valve structure (not shown) and to anchor the prosthetic valve 700 in a native annulus (e.g., a native aortic annulus). The actuators 704 are coupled to the frame 702 and configured to move the frame 702 between a radially expanded configuration and a radially compressed configuration. The actuators 704 are also configured to lock the frame 702 at a desired radial configuration.

The frame 702 of the prosthetic valve 700 can comprise a plurality of pivotably connected struts configured in a manner similar to the frame 12 of the prosthetic valve 10.

As shown in FIGS. 26-29, each actuator 704 can comprise an actuation plate 706 and a locker housing 708. The actuation plate 706 can be coupled to a first end portion (e.g., an inflow end portion) of the frame 702, and the locker housing 708 can be coupled to a second end portion (e.g., an outflow end portion) of the frame 702. The actuation plate 706 can be disposed radially within and selectively axially movable relative to the locker housing 708 in a piston-cylinder like manner, where the actuation plate 706 acts like the piston and the locker housing 708 acts like the cylinder. Thus, when the actuation plate 706 and the locker housing 708 move axially toward each other, the frame 702 radially expands and axially foreshortens, and when the actuation plate 706 and the locker housing 708 move axially away from each other, the frame 702 radially compresses and axially elongates.

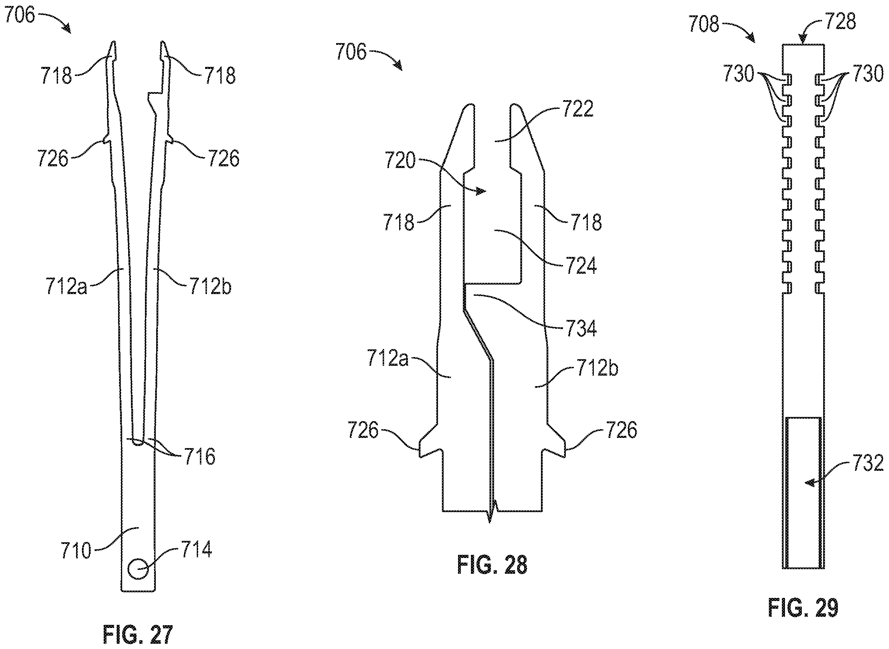

Referring now to FIG. 27, the actuation plate 706 of the actuator 704 can comprise a base portion 710 and a plurality of jaws extending from the base portion 710. For example, the illustrated embodiment includes a first jaw 712a and a second jaw 712b (which are generically or collectively referred to as "the jaws 712"). In other embodiments, the actuation plate 706 can include additional jaws (e.g., 3-4 jaws). The base portion 710 can be configured for coupling the actuation plate 706 to the frame 702. The jaws 712 can be configured to releasably couple the actuation plate 706 to a delivery apparatus, and the delivery apparatus can be configured to move the jaws 712 relative to the locker housing 708, which results in expansion and compression of the frame 702. The jaws 712 can also be configured to selectively engage the locker housing 708, thereby restricting relative movement between the actuation plate 706 and the locker housing 708 and restricting further radial expansion and compression of the frame 702.

The base portion 710 of the actuation plate 706 can be coupled to the frame 702 in various ways. For example, the base portion 710 can comprise an opening 714 configured for receiving a fastener (e.g., a rivet or screw) that couples the actuation plate 706 to the frame 702. In addition or as an alternative to a fastener, the base portion 710 can be coupled to the frame by welding, adhesive, and/or other means for coupling.

Referring to FIG. 27, the jaws 712 of the actuation plate 706 can comprise distal end portions 716 and proximal end portions 718. The distal end portions 716 of the jaws 712 can be coupled to the base portion 710 of the actuation plate 706.

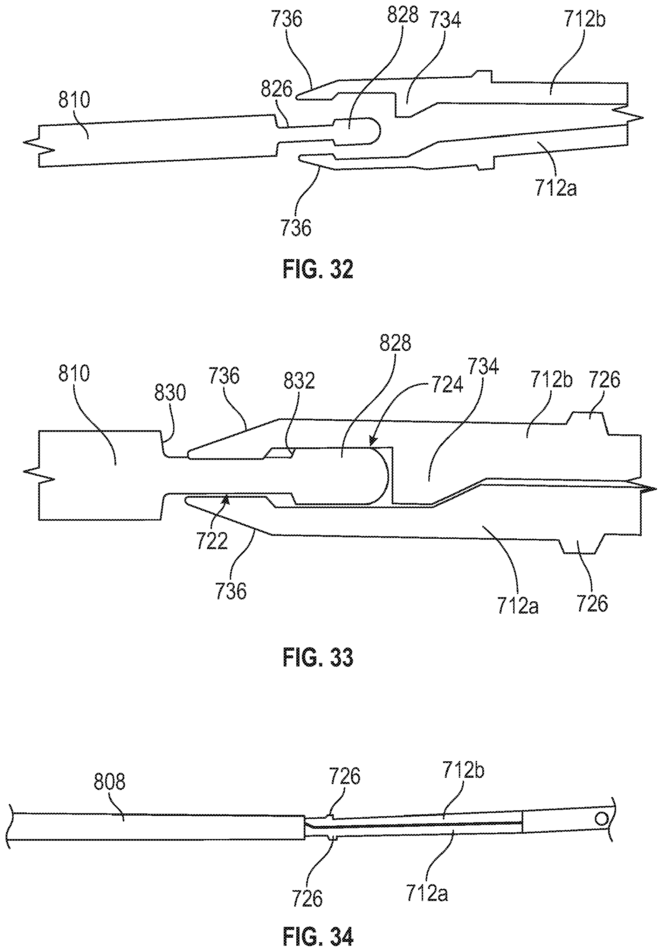

As shown in FIGS. 27-28, the jaws 712 can be configured to be movable relative to each other between an open state (e.g., FIG. 27) and a closed state (e.g., FIG. 28). In the open state, the jaws 712 are spaced apart from each other such that an actuation shaft of a delivery apparatus can be disposed between the jaws 712 (see FIG. 32). In the closed state, the jaws 712 are relatively closer together such that the jaws 712 can engage and secure the actuation shaft between the jaws 712 (see FIG. 33). Additional details regarding the interaction between the actuation plate 706 and the delivery apparatus 800 are provided below.

Referring to FIG. 28, the jaws 712 can be configured such that in the closed state the proximal end portions 718 of the jaws 712 comprises an opening 720. The opening 720 can be configured to receive the actuation shaft of the delivery apparatus and to restrict relative movement between the jaws 712 and the actuation shaft, as shown in FIG. 33. Referring again to FIG. 28, the opening 720 comprise a first portion 722 and a second portion 724 configured for receiving respective portions of the actuation shaft.

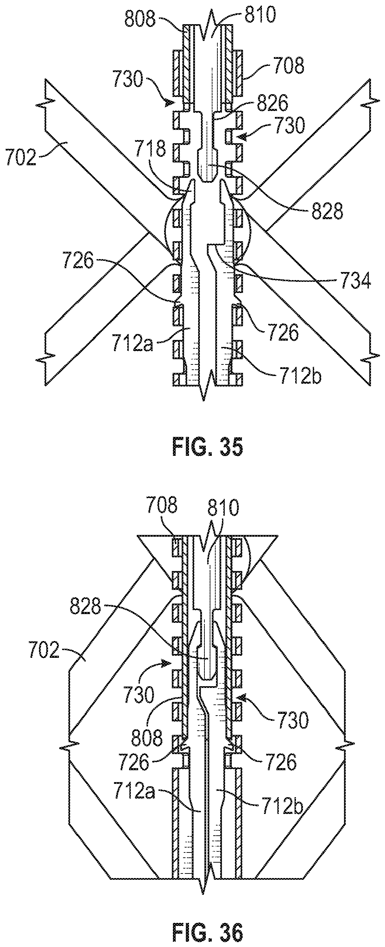

The jaws 712 can comprise projections or tabs 726 extending outwardly therefrom. The jaws 712 can be configured such that the tabs 726 engage the locker housing 708 when the jaws 712 are in the open state, thereby restricting relative movement between the actuation plate 706 and the locker housing 708. The tabs 726 can also act as distal stops for a locking shaft of the delivery apparatus (see FIG. 36), as further described below.

In some embodiments, the jaws 712 of the actuation plate 706 can be configured to be biased toward the open state. This can be accomplished, for example, by forming the jaws 712 of the actuation plate 706 from a flexible, elastic material (e.g., stainless steel, nitinol, and/or other biocompatible metals and/or polymers) and forming (e.g., shape-setting) the jaws 712 in the open state. Additionally or alternatively, a biasing member (e.g., a spring) that is configured to bias the jaws to the open state can be coupled to the jaws.

Referring now to FIG. 29, the locker housing 708 of the actuator 704 can comprise a lumen 728 and one or more slots 730. The lumen 728 of the locker housing 708 can be configured such that the actuation plate 706 and one or more components of the delivery apparatus can be disposed therein. The slots 730 can be configured to receive the tabs 726 of the actuation plate 706 (FIG. 28) when the jaws 712 of the actuation plate 706 are in the open state. When the jaws 712 of the actuation plate 706 are in the open state, the tabs 726 of the actuation plate 706 can extend into the slots 730, thereby restricting relative movement between the actuation plate 706 and the locker housing 708. When the jaws 712 of the actuation plate 706 are in the closed state, the tabs 726 of the actuation plate 706 disengage the slots 730, thereby allowing relative movement between the actuation plate 706 and the locker housing 708. A delivery apparatus can used to selectively engage/disengage the tabs 726 of the actuation plate 706 with/from the slots 730 of the locker housing 708 to move the prosthetic valve 700 between the expanded configuration and a compressed configuration.

The slots 730 can be axially spaced apart relative to each other. In some embodiments, the slots 730 can also be arranged in one or more columns distributed circumferentially around the sleeve. For example, in the illustrated embodiment, the locker housing 708 comprises two columns of slots 730 with each column comprising eleven slots. In other embodiments, the locker housing 708 can comprise less or more than two columns of slots (e.g., 1 or 3-4).

The locker housing 708 can also comprise an opening 732 disposed at the distal end portion of the locker housing 708. The opening 732 of the locker housing 708 can be configured such that the base portion 710 of the actuation plate 706 is exposed from the locker housing 708 when the actuation plate 706 is disposed within the locker housing 708. This can, for example, allow the base portion 710 of the actuation plate 706 to be coupled to the frame 702, as shown in FIG. 26.

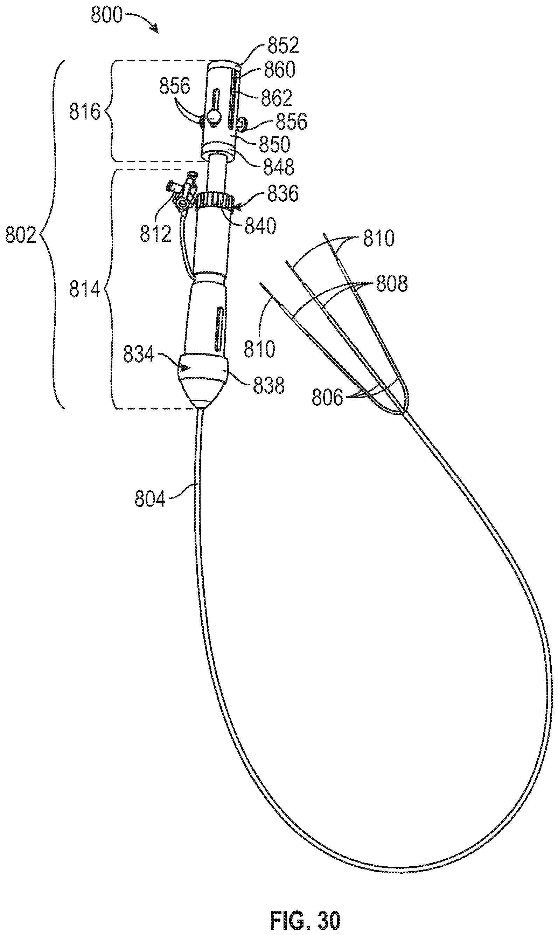

FIG. 30 shows a delivery apparatus 800, according to one embodiment. The prosthetic valve 700 can be coupled to a distal end portion of the delivery apparatus 800 to form a delivery assembly, and the delivery apparatus 800 can be used to implant the prosthetic valve 700 within a patient's body.

Referring to FIGS. 30-31, the delivery apparatus 800 (which can also be referred to as "a valve catheter") can comprise a handle 802, a first shaft 804, one or more second shafts 806, one or more locking shafts 808, one or more actuation shafts 810, and a flushing mechanism 812. The handle 802 can be configured for manipulating the shafts relative to each other. The shafts 804, 806, 808, 810 can be configured for coupling the prosthetic valve 700 to the delivery apparatus 800, for positioning the prosthetic valve 700, and/or for expanding, compressing, and locking the prosthetic valve 700 in a desired radially expanded configuration.

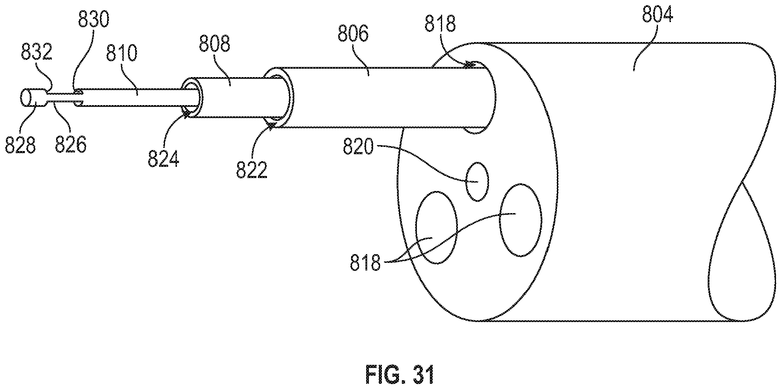

In the some embodiments, the delivery apparatus 800 comprises three of each of the shafts 806, 808, 810 (i.e., one set of shafts 806, 808, 810 for each actuator 704 of the prosthetic valve 700). For purposes of illustration, only one set of the shafts 806, 808, 810 is shown in FIG. 31. In other embodiments, the delivery apparatus 800 can comprise less than three (e.g., 1-2) or more than three (e.g., 4-5) of each of the shafts 806, 808, 810 (e.g., depending on the number of actuators a prosthetic valve includes).

The handle 802 can comprise a first portion 814 and a second portion 816 which are movably coupled together. The shafts 804, 806 can be coupled to the first portion 814 of the handle 802, and the shafts 808, 810 can be coupled to the second portion 816 of the handle 802. As such, the first and second portions 814, 816 of the handle 802 can be used to move the shafts 804, 806 relative to the shafts 808, 810.

Referring now to FIGS. 30-31, the first shaft 804 can extend distally from the first portion 814 of the handle 802 and can comprise one or more lumens for housing other shafts of the delivery apparatus. For example, the first shaft 804 can include one or more first lumens 818 (e.g., three in the illustrated embodiment). Each of the first lumens 818 of the first shaft 804 can be configured to receive a respective second shaft 806. In some embodiments, the first shaft 804 can further comprise one or more additional lumens such as a guidewire lumen 820. The guidewire lumen 820 can be centrally disposed. The first lumen 818 can be disposed radially outwardly relative to the guidewire lumen 820 and spaced circumferentially relative to each other.

The second shafts 806 can extend distally from respective first lumens 818 of the first shaft 804 and can be configured to contact the locker housings 708 of the prosthetic valve 700 (FIG. 26). As such, the second shafts 806 of the delivery apparatus 800 can be used to apply distally-directed forces to the locker housings 708 of the prosthetic valve 700, which can oppose proximally-directed forces applied to the actuation plates 706 of the prosthetic valve 700 by the actuation shafts 810 of the delivery apparatus 800, thereby enabling expansion of the prosthetic valve 700 caused by relative axial movement between the actuation plates 706 and the locker housings 708 of the prosthetic valve 700.

In some embodiments, proximal end portions of the second shafts 806 can be coupled to the first portion 814 of the handle 802, and the second shafts 806 can extend through respective first lumens 818 of the first shaft 804 to the distal end of the first shaft 804. In other embodiments, the second shafts 806 can be relative short tubes that are coupled to the distal end portion of the first shaft 804 but do not extend proximally to the handle 802. In either instance, each of the second shafts 806 can comprise a second lumen 822, which can be configured to receive a respective locking shaft 808.

The locking shafts 808 can extend distally from the second portion 816 of the handle 802 and through the second lumens 822 of respective second shafts 806 (and through respective first lumens 818 of the first shaft 804 prior to extending through the second lumens 822 in embodiments where the second shafts 806 are disposed only at the distal end of the first shaft 804). Each of the locking shafts 808 can comprise a third lumen 824. The third lumens 824 can be configured to receive a respective actuation shaft 810. The third lumens 824 can also be configured such that the locking shafts 808 can be advanced over respective pairs of jaws 712 of the prosthetic valve 700 in order to move and/or retain the jaws 712 of the actuators 704 of the prosthetic valve 700 in the closed state.

The actuation shafts 810 can extend distally from the second portion 816 of the handle 802 and through the third lumens of respective locking shafts 808. The actuation shafts 810 can be releasably coupled to the jaws 712 of the actuators 704 of the prosthetic valve 700 via the locking shafts 808 of delivery apparatus 800. The actuation shafts 810 can therefore be used to radially expand and compress the prosthetic valve 700 as the actuation shafts 810 are moved (together with the locking shafts 808) relative to the second shafts 806.

The distal end portion of the actuation shaft 810 can comprise mating features configured such that the actuation shaft 810 can be releasably coupled to the jaws 712 of the actuator 704 of the prosthetic valve 700. For example, referring to FIG. 31, the distal end portion of each actuation shaft 810 can comprise a neck portion 826 and a head portion 828. The neck portion 826 can comprise radially recessed portions (which can also be referred to as "flats"). As such, the neck portion 826 defines a proximal shoulder 830 at a proximal end of the neck portion 826 and a distal shoulder 832 at the distal end of the neck portion 826. The distal shoulder 832 can define the proximal end of the head portion 828. In this manner, the neck portion 826 and the head portion 828 of the actuation shaft 810 can be disposed in the first portion 722 and the second portion 724 of the opening 720 of the jaws 712, respectively, and thereby releasably couple the actuation shaft 810 to the actuation plate 706, as shown in FIGS. 32-33.

In some embodiments, the prosthetic valve 700 can, for example, be coupled to the distal end portion of the delivery apparatus 800. Referring to FIG. 35, the prosthetic valve 700 can be in the expanded configuration, the jaws 712 of the actuation plate 706 can be in the open state due to their bias toward the open state, and the tabs 726 of the actuation plate 706 can extend into the slots 730 of the locker housing 708. As such, the tabs 726 of the actuation plate 706 engage the locker housing 708 and restrict axial movement of the actuation plate 706 relative to the locker housing 708. This locks the prosthetic valve 700 in an expanded state. With the actuator 704 of the prosthetic valve 700 in the locked configuration, the distal end portion of the actuation shaft 810 can be inserted into the lumen 728 (FIG. 29) of the actuator 704 of the prosthetic valve 700. The actuation shaft 810 can be advanced distally until the neck portion 826 and the head portion 828 of the actuation shaft 810 can be respectively disposed in the first portion 722 and the second portion 724 of the opening 720 of the jaws 712 (see, e.g., FIG. 32).

In some embodiments, the second jaw 712b of the actuation plate 706 can comprise a projection 734 that defines a proximal end of the second portion 724 of the opening 720 of the jaws 712. As the head portion 828 of the actuation shaft 810 is inserted between the jaws 712, the projection 734 of the second jaw 712b can act as a distal stop for the actuation shaft 810. As such, the projection 734 can help axially align the head portion 828 of the actuation shaft 810 with the second portion 724 of the opening 720 and the neck portion 826 with the first portion 722 of the opening 720.

The jaws 712 of the actuation plate 706 can then be moved from the open state to the closed state to releasably couple the actuation plate 706 to the actuation shaft 810 of the delivery apparatus 800. This can be accomplished, for example, by moving the locking shaft 808 distally relative to the actuation shaft 810 and the actuation plate 706. As the locking shaft 808 advances over the jaws 712, the jaws 712 move inwardly toward each other and against the actuation shaft 810, as shown in FIG. 36.

In some embodiments, the proximal end portions 718 of the jaws 712 can comprise ramped outer surfaces 736, as shown in FIG. 33. The ramped outer surfaces 736 can, for example, help the locking shaft 808 slide over the proximal end portions 718 of the jaws 712.

As the jaws 712 move inwardly and into contact with the actuation shaft 810 in the closed state, the tabs 726 of the actuation plate 706 withdraw from the slots 730 of the locker housing 708. The locking shaft 808 can be moved distally relative to the actuation plate 706 until the distal end of the locking shaft 808 abuts the tabs 726 of the actuation plate 706, as shown in FIG. 36. In this manner, the tabs 726 act as distal stops for the locking shaft 808.

With the tabs 726 of the actuation plate 706 disengaged from the slots 730 of the locker housing 708 and the jaws 712 of the actuation plate 706 releasably coupled to the actuation shaft 810, the actuation plate 706 can be moved relative to the locker housing 708, and thus the prosthetic valve 700 can be moved between the expanded configuration and the compressed configuration. This can be accomplished, for example, by moving the actuation shaft 810, the locking shaft 808, and the actuation plate 706 relative to the locker housing 708. As the actuation shaft 810, the locking shaft 808, and the actuation plate 706 move proximally relative to the locker housing 708, the frame 702 of the prosthetic valve 700 radially expands. As the actuation shaft 810, the locking shaft 808, and the actuation plate 706 move distally relative to the locker housing 708 the frame 702 of the prosthetic valve 700 radially contracts.

The second shaft 806 of the delivery apparatus 800 can be used, for example, to provide a force against the locker housing 708 of the prosthetic valve 700 that opposes the force applied to the actuation plate 706 of the prosthetic valve 700 by the actuation shaft 810 of the delivery apparatus 800. For example, the second shaft 806 can be used to apply a distally-directed force on the locker housing 708 when the actuation shaft 810 applying a proximally-directed force on the actuation plate 706. In some embodiments, the distal end of the second shaft 806 can abut the proximal end of the locker housing 708.

When the frame 702 of the prosthetic valve 700 is expanded to the desired configuration, the prosthetic valve 700 can be locked in the desired configuration and released from the delivery apparatus 800. The prosthetic valve 700 can be locked in the desired configuration by moving the locking shaft 808 proximally relative to the actuation shaft 810 and the actuation plate 706 until the distal end of the locking shaft 808 is disposed proximal to the proximal end of the actuation plate 706. This allows the jaws 712 of the actuation plate 706 to move from the closed configuration to the open configuration (due to their bias to the open configuration). As the jaws 712 of the actuation plate 706 open, the tabs 726 of the actuation plate can extend into the slots 730 of the locker housing 708. The engagement between the tabs 726 and the locker housing 708 can restrict further relative movement between the actuation plate 706 and the locker housing 708, and thus lock the prosthetic valve 700 in the desired configuration. When the jaws 712 open, they also disengage the actuation shaft 810, thereby releasing the prosthetic valve 700 from the delivery apparatus 800.

In the event that the tabs 726 of the actuation plate 706 are not axially aligned with the slots 730 of the locker housing 708 when the locking shaft 808 is retracted relative to the actuation shaft 810, the jaws 712 of the actuation plate 706 may not fully open and the actuation plate 706 may remain coupled to the actuation shaft 810. In such instances, the actuation plate 706 can be moved slightly proximally or distally relative to the locker housing 708 via the actuation shaft 810 until the that the tabs 726 of the actuation plate 706 axially align with and expand outwardly into the slots 730 of the locker housing 708. Once the tabs 726 of the actuation plate 706 are disposed in the slots 730, the prosthetic valve 700 is locked in the expanded configuration and released from the actuation shaft 810.

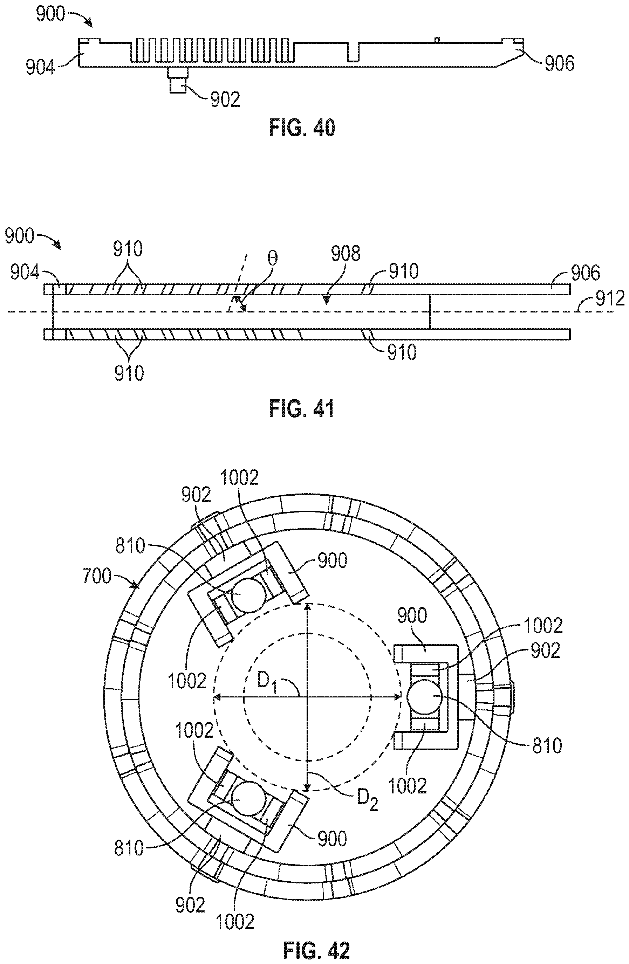

To reduce the likelihood of the tabs 726 of the actuation plate 706 being positioned axially between the slots 730, the spacing between the slots can be reduced. Additionally or alternatively, the slots 730 can be configured such that a first column of slots 730 on a first side of the locker housing 708 (e.g., the slots on the left side of the locking sleeve in the orientation shown in FIGS. 35-36) are axially offset or staggered from a second column of slots 730 on a second side of the locker housing 708 (e.g., the slots on the right side of the locking sleeve in the orientation shown in FIGS. 35-36). An example of a locking sleeve with axially offset slots is shown in FIGS. 42-44.

Referring again to FIG. 30 and as mentioned above, the handle 802 of the delivery apparatus 800 can be configured to manipulate the shafts and move the prosthetic valve 700 to an expanded configuration. The handle 802 can comprise a first portion 814 and a second portion 816 which are movably coupled together. The shafts 804, 806 can be coupled to the first portion 814 of the handle 802, and the shafts 808, 810 can be coupled to the second portion 816 of the handle 802. As such, the first and second portions 814, 816 of the handle 802 can be used to move the shafts 804, 806 relative to the shafts 808, 810.

Referring still to FIG. 30, the first portion 814 of the handle 802 can, in some instances, comprise a steering mechanism 834 and/or an actuation mechanism 836. The steering mechanism 834 can be configured to steer (e.g., flex and/or curve) the distal end portion of the first shaft 804. The steering mechanism 834 can, for example, be used to guide the prosthetic valve 700 through a patient's vasculature (e.g., an aorta) and/or to position the prosthetic valve 700 relative to an implantation location (e.g., a native aortic valve). The actuation mechanism 836 can be configured to move the locking shafts 808 and the actuation shafts 810 relative to the first shaft 804 and the second shafts 806.

In some embodiments, the steering mechanism 834 can comprise one or more pull wires coupled to and extending through the first portion 814 of the handle 802 and through the first shaft 804. The steering mechanism 834 can also comprise one or more actuators (e.g., a rotatable steering knob 838) configured to adjust the tension of the pull wires and thus the flexion of the first shaft 804. In other embodiments, the steering mechanism can comprise electronic components (e.g., motors, circuitry, switches, etc.) configured to actuate the steering mechanism.

In some embodiments, the actuation mechanism 836 can comprise one or more actuators configured to move the locking shafts 808 and the actuation shafts 810 relative to the first shaft 804 and the second shafts 806. For example, in some embodiments, the actuation mechanism can comprise a rotatable actuation knob 840, and the actuation mechanism 836 can be configured such that rotating the actuation knob 840 relative to the first and second portions 814, 816 of the handle 802 results in the locking shafts 808 and the actuation shafts 810 moving axially relative to the first shaft 804 and the second shafts 806. For example, rotating the actuation knob 840 in a first rotational direction (e.g., clockwise) can result in the locking shafts 808 and the actuation shafts 810 moving proximally relative to the first shaft 804 and the second shafts 806. This moves the first and second portions 814, 816 of the handle 802 toward each other. It can also result radial expansion of the prosthetic valve 700 when the prosthetic valve 700 is coupled to the delivery apparatus 800. Rotating the actuation knob 840 in a second rotational direction (e.g., counterclockwise) can result in the locking shafts 808 and the actuation shafts 810 moving distally relative to the first shaft 804 and the second shafts 806. This moves the first and second portions 814, 816 of the handle 802 away from each other. It can also result radial compression of the prosthetic valve 700 when the prosthetic valve 700 is coupled to the delivery apparatus 800. Additional details regarding actuation mechanisms, specifically configuring an actuation mechanism to convert rotational motion of a knob or motor to axial movement between two shafts can be found, for example, in U.S. Patent Nos. 8,652,202, 9,155,619, and 9,867,700, and U.S. Publication No. 2017/0065415.

In other embodiments, the actuation mechanism can comprise electronic components (e.g., motors, circuitry, switches, etc.) configured to actuate the actuation mechanism.

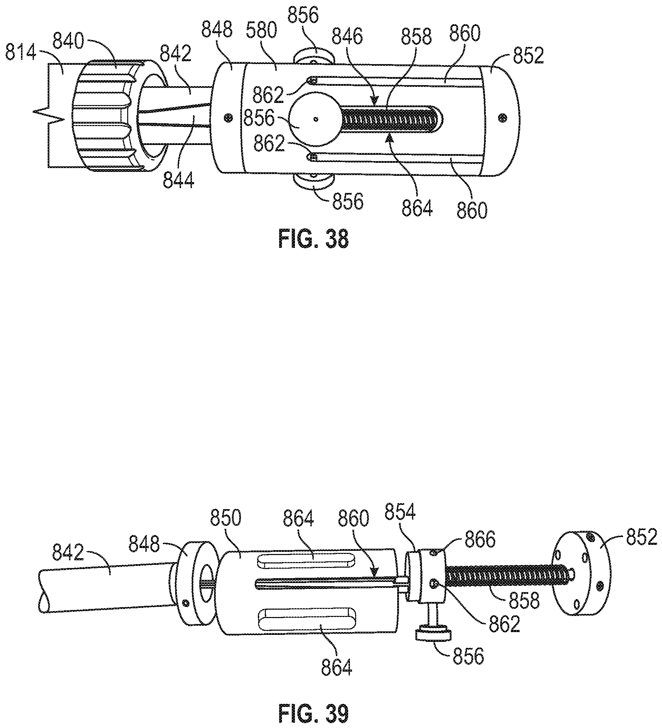

In some embodiments, the actuation mechanism 836 of the handle 802 can comprise a connection member 842 extending between the first and second portions 814, 816 of the handle 802, as shown in FIGS. 38-39. The connection member 842 can, in some instances, comprise a tube or shaft.

Referring to FIGS. 38-39, the actuation mechanism can, in some embodiments, comprise an indicator configured to indicate the expansion of the prosthetic valve. For example, the indicator can be a symbol, markings, display, and/or other type of indicia. For example, in the illustrated embodiment, the actuation mechanism 836 comprises a symbol 844 configured to indicate that the diameter of the prosthetic valve 700 is larger when the first and second portions of the handle move toward each other and that the diameter of the prosthetic valve 700 is smaller when the first and second portions of the handle move away from each other.

Referring still to FIGS. 38-39, the second portion 816 of the handle 802 can, in some embodiments, comprise a locking mechanism 846. The locking mechanism 846 can be configured to selectively control relative movement between the locking shafts 808 and the actuation shafts 810. For example, the locking mechanism 846 can comprise a first mode or state in which the locking shafts 808 and the actuation shafts 810 move together axially and a second mode or state in which the locking shafts 808 move axially relative to the actuation shafts 810. In some embodiments, the locking mechanism 846 can biased to one mode (e.g., the first mode) and selectively movable to the other mode (e.g., the second mode), or vice versa.

Referring to FIG. 38, in addition to the locking mechanism 846, the second portion 816 of the handle 802 comprises a distal cap 848, a main body 850, and a proximal cap 852. The locking mechanism 846 can be disposed within and/or coupled to the main body 850 of the second portion 816 of the handle 802.

The locking mechanism 846 of the second handle portion 816 can comprise one or more slider members 854 (e.g., one in the illustrated embodiment), one or more retention members 856 (e.g., three in the illustrated embodiment) (e.g., handle bolts), and one or more biasing members 858 (e.g., one in the illustrated embodiment).

The distal cap 848 of the handle 802 can be coupled to the connection member 842 (e.g., via fasteners, adhesive, and/or other means for coupling). The distal cap 848 can also be coupled to the distal end portion of the main body 850. The locking shafts 808 and the actuation shafts 810 can extend proximally through the distal cap and into the interior portion of the main body 850. The proximal end portions of the locking shafts 808 can be coupled to the slider member 854. The proximal end portions of the actuation shafts 810 can extend through the slider member 854, extend through the biasing member 858, and be coupled to the proximal cap 852.

The biasing member 858 (e.g., a compression spring) can be disposed axially between the slider member 854 and the proximal cap 852. In this manner, the biasing member 858 biases the slider member 854 distally relative to the proximal cap 852. Since the locking shafts 808 are coupled to the slider member 854 and the actuation shafts 810 are coupled to the proximal cap 852, the biasing member biases the locking shafts 808 distally relative to the actuation shafts 808. This can, for example, help to maintain the locking shafts 808 over the distal end portions of the actuation shafts 810. Thus, when a prosthetic valve 700 is coupled to the delivery apparatus 800, the locking mechanism 846 can be biased to a position in which the locking shafts 808 extend over the jaws 712 of the prosthetic valve 700, which are clamped onto the actuation shafts 810 (see, e.g., FIG. 36). Therefore, the locking mechanism 846 can secure the prosthetic valve 700 to the delivery apparatus 800 in a default position (due to the bias) and can be moved from the default position (by overcoming the bias) to release the prosthetic valve 700 from the delivery apparatus 800.

Referring again to FIGS. 38-39, the locking mechanism 846 can be coupled to the to the second portion 816 of the handle 802 by removing the retention members 856 from the slider member 854 and positioning the slider member 854 and the biasing member 858 within the main body 850 of the handle 802.

In some embodiments, the main body 850 can comprise one or more slots 860 extending axially from the proximal end of the main body 850 toward the distal end of the main body. Each slot 860 can be configured to receive a respective projection 862 that extends radially outwardly from the slider member 854.

With the projections 862 of the slider member 854 circumferentially aligned with the slots 860 of the main body 850, the slider member 854, the biasing member 858, and the proximal cap 852 can be moved distally relative to the main body 850 such that the projections 862 of the slider member 854 are disposed within the slots 860 of the main body 850 and the proximal cap 852 abuts the proximal end of the main body 850. The proximal cap 852 can be coupled to the main body 850 in various ways (e.g., via fasteners, adhesive, and/or other means for coupling). In this manner, the proximal cap 852 can secure the slider member 854 and the biasing member 858 within the main body 850. The projections 862 of the slider member 854 together with the slots 860 of the main body 850 can prevent relative rotational movement between the slider member 854 and the main body 850. The biasing member 858 can urge the slider member 854 toward the distal end portion of the slots 860.

The main body 850 can also comprise one or one or more windows 864 extending axially along the main body 850. Each window 864 of the main body 850 can be configured to receive a respective retention member 856 of the locking mechanism 846. The slider member 854 can comprise one or more bores 866. Each bore 866 can be configured to receive a respective retention member 856. In this manner, a retention member 856 can be inserted through a window 864 of the main body 850 and into a bore 866 of the slider member 854. The retention members 856 and the slider member 854 can be configured such that the extent to which the retention members extend into the bores 866 can be adjusted. For example, the retention members 856 can comprise a shaft with external threads configured to mate with corresponding internal threads of the bores 866.

Referring to FIG. 38, due to the biasing member 858, the retention members 856 and the slider member 854 are biased toward a distal end portion of the main body 850. This locking mechanism 846 can be referred to as "a locked mode" or "a locked position" and corresponds to the relative positioning of the locking shafts 808 and the actuation shafts 810 shown in FIG. 36. The bias toward the locked mode can, for example, help to reduce the likelihood that the prosthetic valve 700 will be inadvertently released from the delivery apparatus 800.

The locking mechanism 846 can be moved from the locked mode to "an unlocked mode" or "an unlocked position" by grasping and moving the retention members 856 proximally relative to the main body 850 with sufficient force to overcome the biasing force of the biasing member 858. This results in the locking shafts 808 moving proximally relative to the actuation shafts 810 to the configuration shown in FIG. 35.

The retention members 856 can be used to lock the position of the slider member 854 (and thus the locking shafts 808) relative to the actuation shafts 810. This can be accomplished by adjusting the retention members 856 relative to the slider member 854 such that the retention members 856 extend radially inwardly beyond the inner diameter of the slider member 854 and press against the actuation shafts 810. In this manner, the retention members 856 act like set screws that frictionally engage the actuation shafts 810 and can restrict relative axial movement between the locking shafts 808 (which are coupled to the slider member 854) and the actuation shafts 810. Thus, in some cases, the retention members 856 can be used as an additional locking mechanism (in addition to the biasing member 858) to prevent the locking shafts 808 from moving proximally relative to the actuation shafts 810, and thereby prevent the prosthetic valve 700 from being released from the delivery apparatus 800. The retention members 856 can also be used, for example, to secure the locking mechanism in the unlocked mode by clamping the slider member 854 relative to the actuation shafts 810 in the unlocked position with sufficient force to resist the force of the biasing member 858. This may be helpful, for example, when coupling the prosthetic valve to the delivery apparatus 800 and/or when releasing the prosthetic valve from the delivery apparatus 800.

In some embodiments, the locking mechanism 846 of the handle 802 can comprise more than one slider member 854. For example, in certain embodiments, the locking mechanism 846 can comprise a slider member 854 for each locking shaft 808 (e.g., three slider members). This can, for example, allow each actuator 704 of the prosthetic valve 700 to be separately coupled to and/or released from the delivery apparatus 800.

The prosthetic valve 700 can be coupled to the delivery apparatus 800 by moving the locking mechanism 846 to the unlocked mode. Referring to FIG. 38, a user can grasp the retention members 856 and move the retention members 856 proximally relative to the main body 850 of the handle 802 so as to overcome the bias of the biasing member 858. This moves the locking shafts 808 proximally relative to the actuation shafts 810 and exposes the neck portion 826 and the head portion 828 at the distal end portion of the actuation shafts 810 (see, e.g., FIG. 31). If desired, the user can secure the locking mechanism 846 in the unlocked mode by adjusting the retention members 856 such that the press against the actuation shafts 810. This prevents the biasing member 858 from urging the slider member 854 distally relative to the main body 850 of the handle 802. Otherwise, the user can simply hold the retention members 856 in the proximal position.

With the locking mechanism 846 in the unlocked mode, the distal end portions of the actuation shafts 810 can be inserted into the proximal end portions of the lumens 728 of the actuators 704 of the prosthetic valve 700. The actuation shafts 810 can be positioned within the openings 720 of the jaws 712 of the actuation plates 706 as shown in FIG. 32. The retention members 856 of the locking mechanism 846 can then be released such that the biasing member 858 can move the slider member 854 distally relative to the main body 850 of the handle 802. This results in the locking shafts 808 moving distally relative to the actuation shafts 810 and the jaws 712 of the actuation plate 706. As such, the locking shafts 808 can move over the jaws 712, which can urge the jaws 712 inwardly to the closed position and couple the actuation plates 706 to the actuation shafts 810 (see, e.g., FIGS. 34 and 36).

In the coupled configuration, radial expansion of the prosthetic valve 700 can be adjusted by actuating the actuation mechanism 836 on the handle 802. This is accomplished to rotating the actuation knob 840 relative to the handle 802, which moves the locking shafts 808, the actuation shafts 810, and the actuation plates 706 axially relative to the locker housings 708, the first shaft 804, and the second shafts 806.

Once the prosthetic valve 700 is in the desired radial configuration, the prosthetic valve 700 can be locked in the radial configuration by moving the locking mechanism again to the unlocked mode. The user can grasp the retention members 856 and move the retention members 856 proximally relative to the main body 850 of the handle 802 so as to overcome the bias of the biasing member 858. This retracts the locking shafts 808 relative to the actuation shafts 810 and the actuation plates 706, thereby allowing the jaws 712 of the actuation plates 706 move to the open position. As a result the tabs 726 of the actuation plates 706 extend radially outwardly into the slots 730 of the locker housings 708 (see, e.g., FIG. 35), which locks the position of the frame 702 of the prosthetic valve 700 in the radial configuration. This also releases the jaws 712 of the actuation plates 706 from the actuation shafts 810, which releases the prosthetic valve 700 from the delivery apparatus 800.

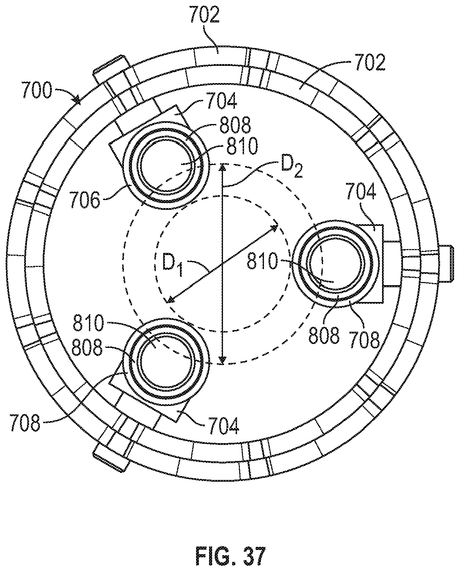

FIGS. 40-41 show a locker housing 900. The locker housing 900 is functionally similar to and can be used as an alternative to the locker housings 708 of the prosthetic valve 700. The locker housing 900 has as a relatively flat, U-shaped cross-section profile taken in a plane perpendicular to a longitudinal axis of the locker housing 900 (see FIGS. 40-42); whereas, the locker housing 708 has a round, circular cross-sectional profile taken in a plane perpendicular to a longitudinal axis of the locker housing 708 (see FIGS. 26 and 37). In some embodiments, the locker housing 900 can have a generally rectangular cross-sectional profile.

Referring to FIG. 40, the locker housing 900 can be coupled to a frame of a prosthetic valve in various ways. For example, the locker housing 900 can comprise one or more coupling elements 902 extending therefrom. The coupling element 902 can be a pin or a projection that is coupled to or integrally formed with the locker housing 900. The coupling element 902 can, in some embodiments, extend through a pivot opening of struts of the frame of a prosthetic valve. In lieu of or in addition to the coupling elements 902, the locker housing 900 can be coupled to the frame of a prosthetic valve with adhesive, sutures, welding and/or other coupling means.

The low-profile configuration of the locker housing 900 can provide several advantages. For example, the locker housing 900 provides additional interior space within a prosthetic heart valve. This can be seen, for example, by comparing FIG. 37 (which shows the prosthetic valve 700 configured with the locker housings 708) and FIG. 42 (which shows the prosthetic valve 700 configured with the locker housings 900). The locker housings 708 can provide the prosthetic valve 700 with an interior diameter D₁; whereas, the locker housings 900 can provide the prosthetic valve 700 with an interior diameter D₂, which is larger than Di. The increased interior space can, for example, provide additional space for the valve structure (e.g., the leaflets) and/or allow the prosthetic valve to be compressed to a smaller radial profile (e.g., for delivery).

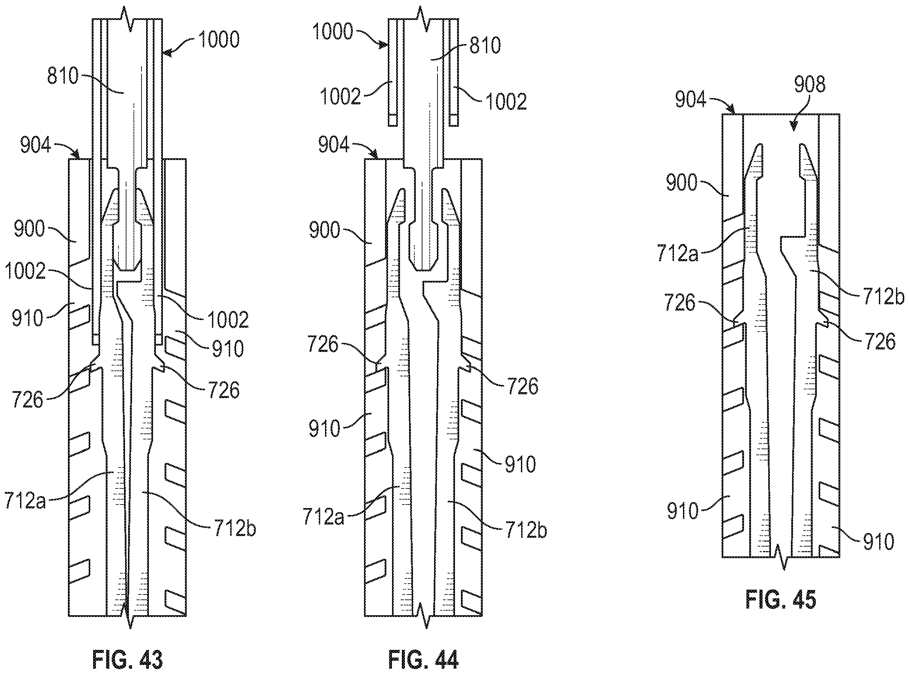

As shown in FIG. 41, the locker housing 900 can comprise a proximal end portion 904, a distal end portion 906, and a central channel 908 extending from the proximal end portion 904 to the distal end portion 906. The central channel 908 can be configured to receive an actuation plate (e.g., the actuation plate 706), as shown in FIGS. 43-45.

Referring to FIGS. 41 and 43-45, the locker housing 900 can also comprise a plurality of slots 910 extending outwardly from the central channel 908. The slots 910 can be configured to receive the tabs 726 of the actuation plate 706 to lock the prosthetic valve at a desired radially expanded state. In some embodiments, the slots 910 on one side of the locker housing 900 can be axially offset or staggered relative to the slots 910 on the other side of the locker housing 900. This can, for example, help to ensure that at least one tab 726 of the actuation plate 706 is disposed within a slot 910 of the locker housing 900 regardless of the axial position of the actuation plate 706 relative to the locker housing 900. The staggered slots 910 can also provide additional locking locations such that the radial expansion of the prosthetic valve can be more finely adjusted.

In some embodiments, the slots 910 can be configured to extend outwardly and distally relative to a longitudinal axis 912 of the locker housing 900 at an angle θ, which is less than 90 degrees. For example, in certain embodiments, the angle θ can be within a range of 45-85 degrees. In particular embodiments, the angle θ can be within a range of 60-80 degrees. In one example, the angle θ can be 70 degrees. The angled slots can, for example, enhance locking force and help to prevent the actuation plate 706 from move distally relative to the locker housing 900, which can reduce the likelihood that the prosthetic valve 700 will inadvertently radially compress.

FIGS. 43-44 also show a locking shaft 1000. The locking shaft 1000 can be configured generally similar to the locking shaft 808, except that the distal end portion of the locking shaft 1000 comprises tines or slats 1002 rather than a hollow tube. The locking shaft 1000 can be used, for example, with the delivery apparatus 800 in lieu of the locking shaft 808.

As shown in FIG. 43, the slats 1002 of the locking shaft 1000 can be configured to extend between the jaws 712 of the actuation plate 706 and the locker housing 900 to retain the jaws 712 in the closed position and thereby couple the actuation plate 706 to the actuation shaft 810. The locking shaft 1000 can be moved together with the actuation shaft 810 and the actuation plate 706 during expansion and/or compression of the prosthetic valve. As shown in FIG. 44, the locking shaft 1000 can also be moved relative to the actuation shaft 810 and the actuation plate 706 to lock the expansion of the prosthetic valve and to release the prosthetic valve from the delivery apparatus.

The features described herein with regard to any example can be combined with other features described in any one or more of the other examples, unless otherwise stated. For example, any one or more of the features of a handle of a delivery apparatus (e.g., the delivery apparatus 200) can be combined with any one or more of the features of another handle of another delivery apparatus (e.g., the delivery apparatus 800). As another example, any of the actuators (e.g., the actuators 16, 104, 404, 604, 704) can be used with any one of the frames.

In view of the many possible embodiments to which the principles of the disclosure may be applied, it should be recognized that the illustrated embodiments are only preferred examples and should not be taken as limiting the scope of the claims. Rather, the scope of the claimed subject matter is defined by the following claims.

## Claims

1. A prosthetic heart valve (10; 100; 400; 600) comprising:
a frame (12; 102) comprising a plurality struts (22; 110) and having an inflow end, an outflow end, and a longitudinal axis extending from the inflow end to the outflow end, wherein the struts (22; 110) are pivotably coupled together such that the frame (12; 102) can pivot between one or more radially compressed states and one or more radially expanded states;
a valve structure (14) disposed within the frame (12; 102), wherein the valve structure (14) comprises a plurality of leaflets (26) configured to allow blood flow through the valve structure (14) from the inflow end of the frame (12; 102) to the outflow end of the frame (12; 102) and to restrict blood flow through the valve structure (14) from the outflow end of the frame (12; 102) to the inflow end of the frame (12; 102); and
an actuator (16; 104; 404; 604) coupled to the frame (12; 102) and configured to move the frame (12; 102) between the one or more radially compressed states and the one or more radially expanded states, wherein the actuator (16; 104; 404; 604) comprises a first portion (128; 414), a second portion (130; 416), a first lumen (124; 423), a second lumen (126; 421), a locking member (118; 418), and a locking element (120; 420), wherein the first lumen (124; 423) and the second lumen (126; 421) extend axially through the first portion (128; 414) and the second portion (130; 416) in a direction parallel to the longitudinal axis of the frame (12; 102), wherein the first lumen (124; 423) and the second lumen (126; 421) are spaced apart from each other and are parallel to each other, wherein the locking member (118; 418) is disposed in the first lumen (124; 423), is fixedly coupled to the first portion (128; 414), and is axially movable relative to a second portion (130; 416), wherein the second lumen (126; 421) is configured to receive an actuation shaft (222; 810) of a delivery apparatus (200; 800),
**characterised in that**
the actuation shaft (222; 810) can be releasably coupled to the first portion (128; 414) and can move axially relative to the second portion (130; 416), and thereby can move the frame (12; 102) between the one or more radially compressed states and the one or more radially expanded states,
and **in that** the locking element (120; 420) is adjustably coupled to the locking member (118; 418) and configured to restrict relative axial movement between the locking member (118; 418) and the second portion (130; 416), and thereby lock the frame (12; 102) in the one or more radially compressed states or the one or more radially expanded states.

2. The prosthetic heart valve (400) of claim 1, wherein the first portion (414) of the actuator (404) is a distal support member (416), and wherein the second portion (416) of the actuator (404) is an intermediate support member (414).

3. The prosthetic heart valve (400) of either claim 1 or claim 2, wherein the locking element (420) is a locking nut, and wherein the locking nut is adjustably coupled to the locking member (418) by a threaded connection.

4. The prosthetic heart valve (400) of any one of claims 1 to 3, wherein the actuator (404) further comprises a third portion, and wherein the third portion is a proximal support member (408; 500).

5. The prosthetic heart valve (10; 100; 400; 600) of any one of claims 1 to 4, wherein the prosthetic heart valve (10; 100; 400; 600) comprises exactly one actuator (16; 104; 404; 604).

6. The prosthetic heart valve (10; 100; 400; 600) of any one of claims 1 to 4, wherein the actuator (16; 104; 404; 604) is one of a plurality of actuators, and wherein the actuators are spaced apart circumferentially relative to each other.

7. The prosthetic heart valve (10; 100; 400; 600) of claim 6, wherein the prosthetic heart valve (10; 100; 400; 600) comprises exactly three actuators.

8. The prosthetic heart valve (400) of any one of claims 1 to 7, wherein the actuator (404) comprises a window (410) disposed between the first lumen (423) and the second lumen (421), and wherein the window (410) is configured to receive a commissure (412) of the leaflets (430) of the valve structure (406).

9. The prosthetic heart valve (400) of any one of claims 1 to 7, wherein the actuator (500) comprises an open slot (502) disposed between the first lumen (423) and the second lumen (421), and wherein the open slot (502) is configured to receive a preassembled commissure (412) of the leaflets (430).

10. The prosthetic heart valve (600) of any one of claims 1 to 9, wherein the frame (602) comprises an apex (606) formed by a pair of struts (22; 110) that is disposed adjacent to the actuator (604), and wherein the pair of struts (22; 110) comprises a first segment (606a) that extends radially outwardly relative to a main body (610) of the frame (606) and relative to the actuator (604) such that there is a radial gap between the first segment (606a) and the actuator (604).

11. The prosthetic heart valve (600) of claim 10, wherein the pair of struts (22; 110) comprises a second segment (606b) extending axially from the first segment (606a) toward the outflow end of the frame (602) and extending radially inwardly from the first segment (606a) so as to radially overlap with an outflow end of the actuator (604).

12. A delivery assembly comprising:
the prosthetic heart valve (10; 100; 400; 600) of any one of claims 1 to 9; and
a delivery apparatus (200; 800) comprising:
an actuation shaft (222; 810), wherein the actuation shaft (222; 810) is configured to be releasably coupled to the first portion (128; 414) of the actuator (16; 104; 404; 604) and to move axially relative to the second portion (130; 416) of the actuator (16; 104; 404; 604), and thereby move the frame (12; 102) between the one or more radially compressed states and the one or more radially expanded states; and
a locking shaft (224; 808), wherein the locking shaft (224; 808) is adjustably coupled to the locking member (118; 418) and configured to move the locking element (120; 420) relative to the locking member (118; 418).

## Patentansprüche

1. Prothetische Herzklappe (10; 100; 400; 600) umfassend:
einen Rahmen (12; 102), der eine Vielzahl von Streben (22; 110) umfasst, und ein Einflussende, ein Ausflussende und eine Längsachse aufweist, die sich vom Einflussende zum Ausflussende erstreckt, wobei die Streben (22; 110) schwenkbar miteinander gekoppelt sind, so dass der Rahmen (12; 102) zwischen einem oder mehreren radial komprimierten Zuständen und einem oder mehreren radial expandierten Zuständen schwenken kann;
eine Klappenstruktur (14), die innerhalb des Rahmens (12; 102) angeordnet ist, wobei die Klappenstruktur (14) eine Vielzahl von Klappensegeln (26) umfasst, die dazu ausgebildet sind, einen Blutfluss durch die Klappenstruktur (14) vom Einflussende des Rahmens (12; 102) zum Ausflussende des Rahmens (12; 102) zu ermöglichen und einen Blutfluss durch die Klappenstruktur (14) vom Ausflussende des Rahmens (12; 102) zum Einflussende des Rahmens (12; 102) einzuschränken; und
eine Betätigungsvorrichtung (16; 104; 404; 604), die mit dem Rahmen (12; 102) gekoppelt und dazu ausgebildet ist, den Rahmen (12; 102) zwischen dem einen oder den mehreren radial komprimierten Zuständen und dem einen oder den mehreren radial expandierten Zuständen zu bewegen, wobei die Betätigungsvorrichtung (16; 104; 404; 604) einen ersten Abschnitt (128; 414), einen zweiten Abschnitt (130; 416), ein erstes Lumen (124; 423), ein zweites Lumen (126; 421), ein Verriegelungsglied (118; 418) und ein Verriegelungselement (120; 420) umfasst, wobei das erste Lumen (124; 423) und das zweite Lumen (126; 421) sich axial durch den ersten Abschnitt (128; 414) und den zweiten Abschnitt (130; 416) in einer Richtung parallel zur Längsachse des Rahmens (12; 102) erstrecken, wobei das erste Lumen (124; 423) und das zweite Lumen (126; 421) voneinander beabstandet und parallel zueinander sind, wobei das Verriegelungsglied (118; 418) in dem ersten Lumen (124; 423) angeordnet ist, fest mit dem ersten Abschnitt (128; 414) gekoppelt ist und relativ zu einem zweiten Abschnitt (130; 416) axial bewegbar ist, wobei das zweite Lumen (126; 421) dazu ausgebildet ist, einen Betätigungsschaft (222; 810) einer Zuführvorrichtung (200; 800) aufzunehmen,
**dadurch gekennzeichnet, dass**
der Betätigungsschaft (222; 810) lösbar mit dem ersten Abschnitt (128; 414) gekoppelt werden kann und sich relativ zu dem zweiten Abschnitt (130; 416) axial bewegen lässt, und dadurch den Rahmen (12; 102) zwischen dem einen oder den mehreren radial komprimierten Zuständen und dem einen oder den mehreren radial expandierten Zuständen bewegen kann,
und dadurch, dass das Verriegelungselement (120; 420) verstellbar mit dem Verriegelungsglied (118; 418) gekoppelt und dazu ausgebildet ist, die relative axiale Bewegung zwischen dem Verriegelungsglied (118; 418) und dem zweiten Abschnitt (130; 416) einzuschränken, und dadurch den Rahmen (12; 102) in dem einen oder den mehreren radial komprimierten Zuständen oder in dem einen oder den mehreren radial expandierten Zuständen zu verriegeln.

2. Prothetische Herzklappe (400) nach Anspruch 1, wobei der erste Abschnitt (414) der Betätigungsvorrichtung (404) ein distales Stützelement (416) ist, und wobei der zweite Abschnitt (416) der Betätigungsvorrichtung (404) ein Zwischenstützelement (414) ist.

3. Prothetische Herzklappe (400) nach Anspruch 1 oder Anspruch 2, wobei das Verriegelungselement (420) eine Verriegelungsmutter ist, und wobei die Verriegelungsmutter durch eine Gewindeverbindung verstellbar mit dem Verriegelungsglied (418) gekoppelt ist.

4. Prothetische Herzklappe (400) nach einem der Ansprüche 1 bis 3, wobei die Betätigungsvorrichtung (404) ferner einen dritten Abschnitt umfasst, und wobei der dritte Abschnitt ein proximales Stützelement (408; 500) ist.

5. Prothetische Herzklappe (10; 100; 400; 600) nach einem der Ansprüche 1 bis 4, wobei die prothetische Herzklappe (10; 100; 400; 600) genau eine Betätigungsvorrichtung (16; 104; 404; 604) umfasst.

6. Prothetische Herzklappe (10; 100; 400; 600) nach einem der Ansprüche 1 bis 4, wobei die Betätigungsvorrichtung (16; 104; 404; 604) eine von einer Vielzahl von Betätigungsvorrichtungen ist, und wobei die Betätigungsvorrichtungen in Umfangsrichtung relativ zueinander beabstandet sind.

7. Prothetische Herzklappe (10; 100; 400; 600) nach Anspruch 6, wobei die prothetische Herzklappe (10; 100; 400; 600) genau drei Betätigungsvorrichtungen umfasst.

8. Prothetische Herzklappe (400) nach einem der Ansprüche 1 bis 7, wobei die Betätigungsvorrichtung (404) ein Fenster (410) umfasst, das zwischen dem ersten Lumen (423) und dem zweiten Lumen (421) angeordnet ist, und wobei das Fenster (410) dazu ausgebildet ist, eine Kommissur (412) der Klappensegel (430) der Klappenstruktur (406) aufzunehmen.

9. Prothetische Herzklappe (400) nach einem der Ansprüche 1 bis 7, wobei die Betätigungsvorrichtung (500) einen offenen Schlitz (502) umfasst, der zwischen dem ersten Lumen (423) und dem zweiten Lumen (421) angeordnet ist, und wobei der offene Schlitz (502) dazu ausgebildet ist, eine vormontierte Kommissur (412) der Klappensegel (430) aufzunehmen.

10. Prothetische Herzklappe (600) nach einem der Ansprüche 1 bis 9, wobei der Rahmen (602) einen Apex (606) umfasst, der durch ein Paar Streben (22; 110) gebildet wird, das neben der Betätigungsvorrichtung (604) angeordnet ist, und wobei das Paar Streben (22; 110) ein erstes Segment (606a) umfasst, das sich relativ zu einem Hauptkörper (610) des Rahmens (606) und relativ zu der Betätigungsvorrichtung (604) radial nach außen erstreckt, so dass ein radialer Spalt zwischen dem ersten Segment (606a) und der Betätigungsvorrichtung (604) besteht.

11. Prothetische Herzklappe (600) nach Anspruch 10, wobei das Paar Streben (22; 110) ein zweites Segment (606b) umfasst, das sich axial von dem ersten Segment (606a) in Richtung des Ausflussendes des Rahmens (602) erstreckt und sich radial von dem ersten Segment (606a) nach innen erstreckt, um sich radial mit einem Ausflussende der Betätigungsvorrichtung (604) zu überlappen.

12. Zuführanordnung, umfassend:
die prothetische Herzklappe (10; 100; 400; 600) nach einem der Ansprüche 1 bis 9; und
eine Zuführvorrichtung (200; 800) umfassend:
einen Betätigungsschaft (222; 810), wobei der Betätigungsschaft (222; 810) dazu ausgebildet ist, lösbar mit dem ersten Abschnitt (128; 414) der Betätigungsvorrichtung (16; 104; 404; 604) gekoppelt zu werden und sich relativ zu dem zweiten Abschnitt (130; 416) der Betätigungsvorrichtung (16; 104; 404; 604) axial zu bewegen, und dadurch den Rahmen (12; 102) zwischen dem einen oder den mehreren radial komprimierten Zuständen und dem einen oder den mehreren radial expandierten Zuständen zu bewegen; und
einen Verriegelungsschaft (224; 808), wobei der Verriegelungsschaft (224; 808) verstellbar mit dem Verriegelungsglied (118; 418) gekoppelt ist und dazu ausgebildet ist, das Verriegelungselement (120; 420) relativ zu dem Verriegelungsglied (118; 418) zu bewegen.

## Revendications

1. Valvule cardiaque prothétique (10 ; 100 ; 400 ; 600) comprenant :
un cadre (12 ; 102) comprenant une pluralité d'entretoises (22 ; 110) et présentant une extrémité d'entrée, une extrémité de sortie et un axe longitudinal s'étendant à partir de l'extrémité d'entrée vers l'extrémité de sortie, les entretoises (22 ; 110) étant accouplées de manière pivotante l'une à l'autre de telle sorte que le cadre (12 ; 102) peut pivoter entre un ou plusieurs états radialement comprimés et un ou plusieurs états radialement déployés ;
une structure de valvule (14) disposée à l'intérieur du cadre (12 ; 102), la structure de valvule (14) comprenant une pluralité de feuillets (26) conçus pour permettre un flux sanguin à travers la structure de valvule (14) à partir de l'extrémité d'entrée du cadre (12 ; 102) vers l'extrémité de sortie du cadre (12 ; 102) et pour restreindre le flux sanguin à travers la structure de valvule (14) à partir de l'extrémité de sortie du cadre (12 ; 102) vers l'extrémité d'entrée du cadre (12 ; 102) ; et
un actionneur (16 ; 104 ; 404 ; 604) accouplé au cadre (12 ; 102) et conçu pour déplacer le cadre (12 ; 102) entre ledit un ou lesdits plusieurs états radialement comprimés et ledit un ou lesdits plusieurs états radialement déployés, l'actionneur (16 ; 104 ; 404 ; 604) comprenant une première partie (128 ; 414), une deuxième partie (130 ; 416), une première lumière (124 ; 423), une deuxième lumière (126 ; 421), un élément de verrouillage (118 ; 418) et un élément de verrouillage (120 ; 420), la première lumière (124 ; 423) et la deuxième lumière (126 ; 421) s'étendant axialement à travers la première partie (128 ; 414) et la deuxième partie (130 ; 416) dans une direction parallèle à l'axe longitudinal du cadre (12 ; 102), la première lumière (124 ; 423) et la deuxième lumière (126 ; 421) étant écartées l'une de l'autre et étant parallèles l'une à l'autre, l'élément de verrouillage (118 ; 418) étant disposé dans la première lumière (124 ; 423), étant accouplé de manière fixe à la première partie (128 ; 414) et étant mobile axialement par rapport à une deuxième partie (130 ; 416), la deuxième lumière (126 ; 421) étant conçue pour recevoir un arbre d'actionnement (222 ; 810) d'un appareil de pose (200 ; 800),
**caractérisée en ce que** l'arbre d'actionnement (222 ; 810) peut être accouplé de manière amovible à la première partie (128 ; 414) et peut se déplacer axialement par rapport à la deuxième partie (130 ; 416) et peut ainsi déplacer le cadre (12 ; 102) entre ledit un ou lesdits plusieurs états radialement comprimés et ledit un ou lesdits plusieurs états radialement déployés et **en ce que** l'élément de verrouillage (120 ; 420) est accouplé de manière ajustable à l'élément de verrouillage (118 ; 418) et conçu pour restreindre un mouvement axial relatif entre l'élément de verrouillage (118 ; 418) et la deuxième partie (130 ; 416) et verrouiller ainsi le cadre (12 ; 102) dans ledit un ou lesdits plusieurs états radialement comprimés ou ledit un ou lesdits plusieurs états radialement déployés.

2. Valvule cardiaque prothétique (400) selon la revendication 1, la première partie (414) de l'actionneur (404) étant un élément support distal (416) et la deuxième partie (416) de l'actionneur (404) étant un élément support intermédiaire (414).

3. Valvule cardiaque prothétique (400) selon la revendication 1 ou la revendication 2, l'élément de verrouillage (420) étant un écrou de verrouillage et l'écrou de verrouillage étant accouplé de manière ajustable à l'élément de verrouillage (418) par une connexion filetée.

4. Valvule cardiaque prothétique (400) selon l'une quelconque des revendications 1 à 3, l'actionneur (404) comprenant en outre une troisième partie et la troisième partie étant un élément support proximal (408 ; 500).

5. Valvule cardiaque prothétique (10 ; 100 ; 400 ; 600) selon l'une quelconque des revendications 1 à 4, la valvule cardiaque prothétique (10 ; 100 ; 400 ; 600) comprenant exactement un actionneur (16 ; 104 ; 404 ; 604).

6. Valvule cardiaque prothétique (10 ; 100 ; 400 ; 600) selon l'une quelconque des revendications 1 à 4, l'actionneur (16 ; 104 ; 404 ; 604) étant un élément parmi une pluralité d'actionneurs et les actionneurs étant écartés de manière circonférentielle les uns par rapport aux autres.

7. Valvule cardiaque prothétique (10 ; 100 ; 400 ; 600) selon la revendication 6, la valvule cardiaque prothétique (10 ; 100 ; 400 ; 600) comprenant exactement trois actionneurs.

8. Valvule cardiaque prothétique (400) selon l'une quelconque des revendications 1 à 7, l'actionneur (404) comprenant une fenêtre (410) disposée entre la première lumière (423) et la deuxième lumière (421) et la fenêtre (410) étant conçue pour recevoir une commissure (412) des feuillets (430) de la structure de valvule (406).

9. Valvule cardiaque prothétique (400) selon l'une quelconque des revendications 1 à 7, l'actionneur (500) comprenant une fente ouverte (502) disposée entre la première lumière (423) et la deuxième lumière (421) et la fente ouverte (502) étant conçue pour recevoir une commissure préassemblée (412) des feuillets (430).

10. Valvule cardiaque prothétique (600) selon l'une quelconque des revendications 1 à 9, le cadre (602) comprenant un sommet (606) formé par une paire d'entretoises (22 ; 110) qui est disposée de manière adjacente à l'actionneur (604) et la paire d'entretoises (22 ; 110) comprenant un premier segment (606a) qui s'étend radialement vers l'extérieur par rapport à un corps principal (610) du cadre (606 ) et par rapport à l'actionneur (604) de telle sorte qu'il existe un interstice radial entre le premier segment (606a) et l'actionneur (604).

11. Valvule cardiaque prothétique (600) selon la revendication 10, la paire d'entretoises (22 ; 110) comprenant un deuxième segment (606b) s'étendant axialement à partir du premier segment (606a) vers l'extrémité de sortie du cadre (602) et s'étendant radialement vers l'intérieur à partir du premier segment (606a) de façon à chevaucher radialement une extrémité de sortie de l'actionneur (604).

12. Ensemble de pose comprenant :
la valvule cardiaque prothétique (10 ; 100 ; 400 ; 600) selon l'une quelconque des revendications 1 à 9 ; et
un appareil de pose (200 ; 800) comprenant :
un arbre d'actionnement (222 ; 810), l'arbre d'actionnement (222 ; 810) étant conçu pour être accouplé de manière amovible à la première partie (128 ; 414) de l'actionneur (16 ; 104 ; 404 ; 604) et pour se déplacer axialement par rapport à la deuxième partie (130 ; 416) de l'actionneur (16 ; 104 ; 404 ; 604) et déplacer ainsi le cadre (12 ; 102) entre ledit un ou lesdits plusieurs états radialement comprimés et ledit un ou lesdits plusieurs états radialement déployés ; et
un arbre de verrouillage (224 ; 808), l'arbre de verrouillage (224 ; 808) étant accouplé de manière ajustable à l'élément de verrouillage (118 ; 418) et conçu pour déplacer l'élément de verrouillage (120 ; 420) par rapport à l'élément de verrouillage (118 ; 418).
